# EUROPEAN PATENT SPECIFICATION

(11) **EP 2 117 563 B1**
(45) Date of publication and mention of the grant of the patent: **10.10.2012**
(21) Application number: 08712676.9
(22) Date of filing: 07.02.2008
(51) Int. Cl.: A61K 31/704, A61K 31/7056, A61K 31/706, A61K 31/7064, A61K 9/00, A61K 9/19, A61K 47/26, A61P 31/12

(54) **Amidinoanthracycline antibiotics for use in the treatment of viral infections**
Amidinanthracyclin-Antibiotika zur Verwendung bei der Behandlung von Virusinfekten
Antibiotiques d'anthracycline pour le traitement d'infections virales

(30) Priority: 07.02.2007 PL 38171407
(43) Date of publication of application: 18.11.2009
(73) Proprietor: Instytut Biotechnologii I Antybiotykow, 02-516 Warszawa (PL); Instytut Biochemii I Biofizyki, 02-106 Warszawa (PL)
(72) Inventor: OSZCZAPOWICZ Irena, 00-096 Warszawa (PL); BOGUSZEWSKA-CHACHULSKA, Anna, 02-109 Warszawa (PL); KRAWCZYK, Mariusz, 05-090 Raszyn (PL); LUKAWSKA, Malgorzata, 02-778 Warszawa (PL)
(74) Representative: Kiewlicz, Zofia
(86) International application number: PCT/PL2008/000010
(87) International publication number: WO 2008/097112

(56) References cited:
- WO-A1-2007/075092
- PL-B1- 186 762
- BOROWSKI P ET AL: "NUCLEOTIDE TRIPHOSPHATASE/HELICASE OF HEPATITIS C VIRUS AS A TARGET FOR ANTIVIRAL THERAPY" ANTIVIRAL RESEARCH, ELSEVIER SCIENCE BV., AMSTERDAM, NL, vol. 55, no. 3, 1 January 2002 (2002-01-01), pages 397-412, XP008056992 ISSN: 0166-3542 cited in the application
- CHANDRA, P. ET AL: "Specific inhibition of DNA-polymerases from RNA tumor viruses by some new daunomycin derivatives" FEBS LETTERS, vol. 21, no. 3, 1972, pages 264-268, XP002482883 ISSN: 0014-5793
- BACHUR N R ET AL: "ANTHRACYCLINE ANTIBIOTIC BLOCKADE OF SV40 T ANTIGEN HELICASE ACTION" BIOCHEMICAL PHARMACOLOGY, PERGAMON, OXFORD, GB, vol. 55, no. 7, 1 April 1998 (1998-04-01), pages 1025-1034, XP008056970 ISSN: 0006-2952
- AJITO, KEIICHI ET AL: "Inhibition of human immunodeficiency virus -associated reverse transcriptase by 14-O-acyladriamycins" JOURNAL OF ANTIBIOTICS, vol. 42, no. 4, 1989, pages 611-619, XP009101119 ISSN: 0021-8820
- MINASYAN SH KH ET AL: "Influence of rubomycin, carminomycin, doxorubicin, and their semisynthetic derivatives on in vitro DNA synthesis" KHIMIKO-FARMATSEVTICHESKII ZHURNAL, MOSCOW, RU, vol. 23, no. 10, 1 January 1989 (1989-01-01), pages 791-794, XP009101048 ISSN: 0023-1134

## Description

The present invention relates to the medical use of anthracycline antibiotics derivatives.

These derivatives are represented by general formula 1 and 2, wherein R¹ is hydrogen or methoxy group, R² is hydrogen or hydroxy group, R³ is hydroxy group in an axial or equatorial orientation, R⁴ and R⁵ are hydrogen or methyl group, R⁶ is 1-phenylethyl group or R⁵ and R⁶ together with a nitrogen atom are N,N-diethyl, N,N-dipropyl, N,N-dibutyl group or R⁵ and R⁶ together with a nitrogen atom form N,N-1",4"-tetramethylene, N,N-3"-oxa-1",5"-pentamethylene, N,N-1",5"-pentamethylene, N,N-1",6"-hexamethylene, N,N-1",7"-heptamethylene, N,N-3"-methylaza-1",5"-pentamethylene or 1-indolinyl group, and when X is HCl molecule, the compound is hydrochloride of a derivative of general formula 1 or if X is 0, the compound is a derivative - a free base of general formula 1, which derivatives as active substances are used for preparation of a medicine for treatment of viral infections.

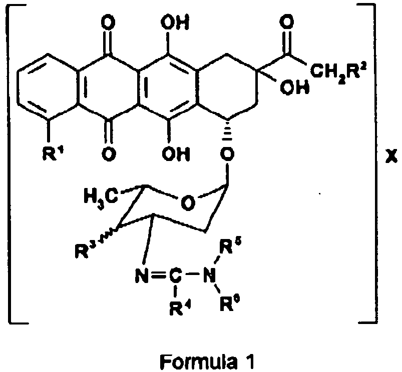

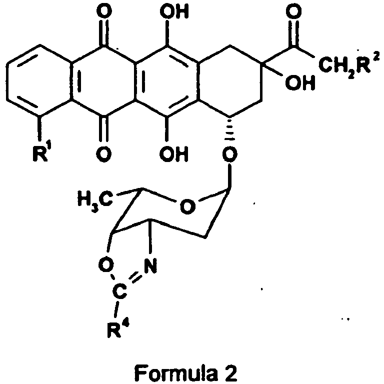

HCV infection is, at present, the second most frequent cause of acute or chronic hepatitis, after infection with hepatitis B virus [World Health Organisation (WHO), 2007]. About 3% of world population is infected by that virus (180 million, WHO, 2007), whereas in Poland more than 2%. At present, a number of deaths per year caused by the virus all over the world amounts to 8000-12000, and in the next few years it will probably double.

HCV infection in about 80% of cases results in chronic hepatitis, and in 4 to 20% patients within 5 to 20 years ends in cirrhosis, which involves permanent treatment and results in a permanent disability; in the case of 1-5% chronically infected patients primary liver cancer develops (Brass V. et al., International J. Medicinal Sciences 3, 29-34, 2006). Because of HCV interactions with immunological system during chronic infections other diseases may occur, being an extrahepatic HCV manifestation. There is no vaccine against HCV due to high variability of viral RNA genome, and thus of viral proteins, particularly envelope proteins.

Methods of treatment of chronic hepatitis C evolve very fast. At first, monotherapy with interferon alpha (IFN) was used, whereas at the end of 90-ties a more effective combined therapy with IFN and ribavirin was introduced, and further modification of therapy consisted in using pegylated interferon. At present, a valid treatment scheme is a combined therapy with pegylated interferon alpha-2a or alpha-2b and ribavirin, the dosage being dependent on the HCV genotype. In the case of occurrence of distinct adverse reactions, ineffective treatment, or recurrence of virus replication as well as when treating patients under 18 years old most often a monotherapy is used with other interferons, such as natural interferon alpha-n3 or other preparations, e.g. comprising interferon alpha-2a in a formulation providing prolonged action, optionally combined with ribavirin.

Prior to the treatment the HCV genotype in a patient should be determined, as its type defines a relevant treatment method, and in cases of infections caused by genotypes 1, 4, 5 or 6- also the concentration of HCV RNA (a degree of viremia) has to determined. The treatment is considered to be effective if after 24 weeks upon the end of therapy, no HCV RNA is detected in blood.

If the pharmacological treatment fails, the only possible therapy method is liver transplantation, considered as one of the most difficult surgical procedures because of liver position and its blood supply.

As until now no fully effective and economic drug against various genotypes exists, there is a need for search of new, more effective and economic therapies against HCV. The search is directed to improve the present treatment standard by employing new interferons (interferon beta-1c) or more suitable forms of that drug, such as prolonged-action interferons or new derivatives of ribavirin exhibiting weaker adverse effects.

Another direction of research pertains to a possibility of interference into the HCV replication cycle. Important elements of such action can be different viral proteins - the NS3 protease/RNA helicase/NTP-ase or the RNA-dependent RNA polymerase (Huang M. and Deshpande M., Expert Rev. Antiinfect. Ther. 2, 375-388, 2004).

The NS3 helicase of HCV seems to be one of the best targets of activity of low-molecular inhibitors as an enzyme indispensable for viral multiplication (Lam A.M. and Frick D.N., J. Virology 80, 404-411, 2006). The helicase activity is necessary at the most important stages of viral life cycle, such as replication and translation. Inhibition of helicase activity and increase of double-stranded RNA level will result in stimulation of antiviral cell response (Gordon C.P. and Keller P.A., J. Med. Chem. 48, 1-20, 2005).

In the prior art, among many examined compound types, for which antiviral activity was proved, there were also anthracycline antibiotics. It has been found, for example, that these antibiotics inhibit activity of enzymes and/or replication of Human immunodeficiency virus (HIV) of (+)RNA genome. From U.S. Patent No. 5.003.055 a group of daunorubicin derivatives, inhibiting activity of HIV reverse transcriptase, is known. It has been also proved that doxorubicin selectively inhibits replication of HIV in macrophages, but not in lymphocytes (Bergamini A. et al., AIDS Res. Hum. Retroviruses, 8, 1239-1247, 1992), probably acting by blocking reverse transcriptase reaction. In a similar manner, daunorubicin much more effectively inhibits replication of the virus in monocytes than in lymphocytes T (Filion A. et al., Clin. Invest. Med. 16, 339-347, 1993). Recent studies have proved that bis-anthracycline derivative denoted as WP631, including molecules built from two daunorubicin moieties connected by a p-xylene group, inhibits HIV replication in lymphocytes (PBMC cells) through inhibition of transactivation of viral protein Tat. Because of low therapeutic index, the compound can serve only as a substrate for synthesis of new derivatives (Kutsch W. et al., Antimicrobial Agents and Chemotherapy 48, 1652-1663, 2004).

Moreover, for several anthracycline antibiotics such as daunorubicin, doxorubicin, epirubicin, mitoxantrone and nogalamycin, Borowski et al. have proved a significant antihelicase activity (IC₅₀ values are 57.0, 5.0, 0.75, 6.7 and 0.1 µM, respectively) but the authors emphasized the high cytotoxicity of these compounds (Borowski P. et al., Antiviral Res. 55, 397-412, 2002).

From Patent Application No.WO2007/075092A1 are known the compounds wherein the amino group in position 3' of anthracycline antibiotic of formula 1 is substituted, or amidrazoneanthracyclines in a case wherein substituent R₃ and/or R₄ of anthracycline antibiotic of formula 1/ WO2007/075092/ is dimethylformamidine group. (" The chemistry of Amidines and Imidates". S. Patal and all. 1991). The compounds are used for inhibiting hepatitis C virus replication. Application concerns the structures that are different from amidinoanthracyclines disclosed in present patent application.

From EP Patent No. 1721614A1 an antiviral activity of epirubicin hydrochloride is also known but this antibiotic exhibit high toxicity.

From the prior art, the above-mentioned amidinoanthracyclines of general formula 1 and 2, wherein R¹, R², R³, R⁴, R⁵, R⁶ and X have the above-given meaning, were described in PL Patent No. 186762 and in PL Patent Applications Nos. P. 372208 and P. 378888. These derivatives, prepared by transformation of the amino group at 3' position of anthracycline antibiotic to an amidino group exhibit very advantageous biological properties, such as high cytotoxic activity, considerable, up to 60-fold decrease of toxicity as compared to the parent anthracycline antibiotics, including cardiotoxicity, and moreover, contrary to the parent antibiotics, an ability to overcome a barrier of resistance of tumour cells (Wasowska M. et al., Anticancer Res., 26, 2009-2012, 2005 and Wasowska M. et al, Anticancer Res. 25, 2043-2048, 2006).

The inventors in their present studies, have unexpectedly found, that amidinoanthracyclines, derivatives of such antibiotics as daunorubicin, doxorubicin, epldaunorubicin and epidoxorubicin, represented by general formula 1 or 2, wherein R¹, R², R³, R⁴, R⁵, R⁶ and X have the above-given meaning, appear to be also strong inhibitors of helicase of hepatitis C virus (HCV), and considerably stronger that the above-mentioned parent anthracycline antibiotics. These results made possible to introduce new medical applications of the above-mentioned derivatives of general formulae 1 or 2, according to the invention.

It should be pointed out, that presence of the amidino group in a given compound sometimes results in an antiviral activity. Such compounds having a broad range of antiviral action include, *inter alia*, amidinomycin, netropsin and distamycin (The chemistry of amidines and imidates, edited by S. Patai, John Wiley & Sons, London, New York, Toronto, pp. 255-282, 1975), as well as N-phenyl derivatives, e.g. N'-(4-{[methyl-(2-propionyl)-aminol-methyl}-phenyl)-2-tiophene-carboxyiminoamide (FR Patent Application No. 2601053-A1) or cis-2,6-(4,8-dioxa-bicyclo-[3.3.0]octylene-bis-[4-(4-amidinobenzyl-carbamoyl)-piperazinecarboxylate (U.S.Patent No.6211228-B1).

Because of that, a crucial problem solved by the present invention is an evidence of antihelicase activity of derivatives represented by general formula 1 or 2, containing the amidino group in position 3' and leading to a new use of these derivatives.

A main aspect of the invention is a medical use of anthracycline antibiotics derivatives of general formulae 1 or 2, wherein R¹ is hydrogen or methoxy group, R² is hydrogen or hydroxy group, R³ is hydroxy group in an axial or equatorial orientation, R⁴ and R⁵ are hydrogen or methyl group, R⁶ is 1-phenylethyl, or R⁵ and R⁶ together with a nitrogen atom are N,N-diethyl, N,N-dipropyl or N,N-dibutyl group, or R⁵ and R⁶ together with a nitrogen atom form N,N-1",4"-tetramethylene, N,N-3"-oxa-1",5"-pentamethylene, N,N-1",5"-pentamethylene, N,N-1",6"-hexamethylene, N,N-1",7"-heptamethylene, N,N-3"-methylaza-1",5"-pentamethylene or 1-indolinyl group and if X is HCl molecule, the compound is hydrochloride of a derivative of general formula 1 or if X is 0, the compound is a derivative in a free base form of general formula 1, wherein these derivatives are used as active ingredients for preparation of a medicine for treatment of viral infections, caused by hepatitis C virus (HCV).

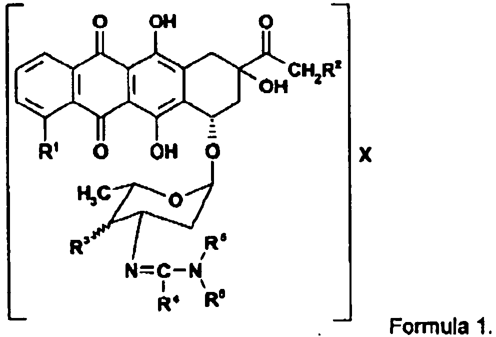

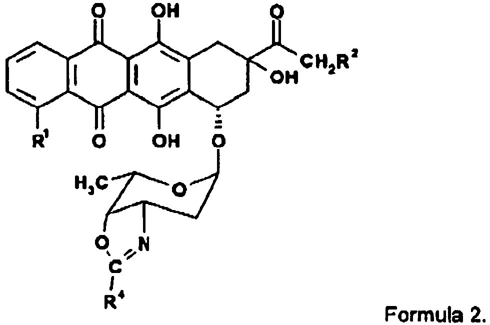

In studies of antihelicase activity, for the purpose of a medical application according to the invention, derivatives, represented by general formula 1, wherein R¹, R², R³. R⁴ and R⁵, R⁶ and X have the above-given meaning, are selected from the group, consisting of :
3'-deamino-3'-(N,N-1",4"-tetramethyleneformamidino)-daunorubicin hydrochloride **(1)**,
3'-deamino-3'-(N,N-1",4"-tetramethyleneformamidino)-daunorubicin **(2)**,
3'-deamino-3'-(N,N-3"-oxa-1",5"-pentamethyleneformamidino)-daunorubicin hydrochloride (3),
3'-deamino-3'-(N,N-3"-oxa-1",5"-pentamethyleneformamidino)-daunorubicin **(4)**,
3'-deamino-3'-(N,N-1",5°-pentamethyleneformamidino)-daunorubicin hydrochloride **(5)**,
3'-deamino-3'-(N,N-1",5"- pentamethyleneformamidino)-daunorubicin **(6)**,
3'-deamino-3'-(N,N-1",6"-hexamethyleneformamidino)-daunorubicin hydrochloride **(7)**,
3'-deamino-3'-(N,N-3"-methylaza-1",5"-pentamethyleneformamidino)-daunorubicin hydrochloride **(8)**,
3'-deamino-3'-(N,N-3"methylaza-1",5"-pentamethyleneformamidino)-daunorubicin **(9)**,
3'-deamino-3'-(N,N-o-ethylenephenyleneformamidino)-daunorubicin hydrochloride **(10)**,
3'-deamino-3'-(N,N-o-ethylenephenyleneformamidino)-daunorubicin **(11)**,
3'-deamino-3'-(N,N-diethylformamidino)-daunorubicin hydrochloride **(12)**,
3'-deamino-3'-(N,N-diethylformamidino)-daunorubicin **(13)**,
3'-deamino-3'-(N,N-dipropylformamidino)-daunorubicin hydrochloride **(14)**,
3'-deamino-3'-(N,N-dipropylformamidino)-daunorubicin **(15)**,
3'-deamino-3'-(N,N-dibutylformamidino)-daunorubicin hydrochloride **(16)**,
3'-deamino-3'-(N,N-dibutylformamidino)-daunorubicin **(17)**,
3'-deamino-3'-(N,N-1",4"-tetramethyleneacetamidino)-daunorubicin hydrochloride **(18)**,
3'-deamino-3'-(N,N-1",4"-tetramethyleneacetamidino)-daunorubicin **(19)**,
3'-dearnino-3'-(N,N-3"-oxa-1",5"-pentamethyleneacetamidino)-daunorubicin hydrochloride **(20)**,
3'-deamino-3'-(N,N-3"-oxa-1 °,5°-pentamethyleneacetamidino)-daunorubicin **(21)**,
3'-deamino-3'-(N,N-1",5"-pentamethyleneacetamidino)-daunorubicin hydrochloride **(22)**,
3'-deamino-3'-(N,N-1",5"-pentamethyleneacetamidino)-daunorubicin **(23)**,
3'-deamino-3'-(N,N-1",6"-hexamethyleneacetamidino)-daunorubicin hydrochloride **(24)**,
3'-deamino-3'-(N,N-3"-methylaza-1",5"-pentamethyleneacetamidino)-daunorubicin hydrochloride **(25)**,
3'-deamino-3'-(N,N-3"-methylaza-1",5"-pentamethyleneacetamidino)-daunorubicin **(26)**,
3'-deamino-3'-(N,N-o-ethylenephenyleneacetamidino)-daunorubicin hydrochloride **(27)**,
3'-deamino-3'-(N,N-o-ethylenephenyleneacetamidino)-daunorubicin **(28)**,
3'-deamino-3'-(N,N-diethylacetamidino)-daunorubicin hydrochloride **(29)**,
3'-deamino-3'-(N,N-diethylacetamidino)-daunorubicin **(30)**,
3'-deamino-3'-(N,N-dipropylacetamidino)-daunorubicin hydrochloride **(31)**,
3'-deamino-3'-(N,N-dipropylacetamidino)-daunorubicin **(32)**,
3'-deamino-3'-(N,N-dibutylacetamidino)-daunorubicin hydrochloride **(33)**,
-3'-deamino-3'-(N,N-dibutylacetamidino)-daunorubicin **(34)**,
3'-deamino-3'-(N,N-1",4"-tetramethyleneformamidino)-doxorubicin hydrochloride **(35)**,
3'-deamino-3'-(N,N-1",4"-tetramethyleneformamidino)-doxorubicin **(36)**,
3'-deamino-3'-(N,N-3"-oxa-1",5"-pentamethyleneformamidino)-doxorubicin hydrochloride **(37)**,
3'-deamino-3'-(N,N-3"-oxa-1",5"-pentamethyleneformamidino)-doxorubicin **(38)**,
3'-deamino-3'-(N,N-1",5"-pentamethyleneformamidino)-doxorubicin hydrochloride **(39)**,
3'-deamino-3'-(N,N-1",5"-pentamethyleneformamidino)-doxorubicin **(40)**,
3'-deamino-3'-(N,N-1",6"-hexamethyleneformamidino)-doxorubicin hydrochloride **(41)**,
3'-deamino-3'-(N,N-3"-methylaza-1",5"-pentamethyleneformamidino)-doxorubicin hydrochloride **(42)**,
3'-deamino-3'-(N,N-3"-methylaza-1",5"-pentamethyleneformamidino)-doxorubicin **(43)**,
3'-deamino-3'-(N,N-o-ethylenephenyleneformamidino)-doxorubicin hydrochloride **(44)**,
3'-deamino-3'-(N,N-o-ethylenephenyleneformamidino)-doxorubicin **(45)**,
-3'-deamino-3'-(N,N-diethylformamidino)-doxorubicin hydrochloride **(46)**,
3'-deamino-3'-(N,N-diethylformamidino)-doxorubicin **(47)**,
3'-deamino-3'-(N,N-dipropylformamidino)-doxorubicin hydrochloride **(48)**,
3'-deamino-3'-(N,N-dipropylformamidino)-doxorubicin **(49)**,
3'-deamino-3'-(N,N-dibutylformamidino)-doxorubicin hydrochloride **(50)**,
3'-deamino-3'-(N,N-dibutylformamidino)-doxorubicin **(51)**,
3'-deamino-3'-(N,N-1",4"-tetramethyleneacetamidino)-doxorubicin hydrochloride **(52)**,
3'-deamino-3'-(N,N-1",4"-tetramethyleneacetamidino)-doxorubicin **(53)**,
3'-deamino-3'-(N,N-3"-oxa-1",5"-pentamethyleneacetamidino)-doxorubicin hydrochloride **(54)**,
3'-deamino-3'-(N,N-3"-oxa-1",5"-pentamethyleneacetamidino)-doxorubicin **(55)**,
3'-deamino-3'-(N,N-1",5"-pentamethyleneacetamidino)-doxorubicin hydrochloride **(56)**,
3'-deamino-3'-(N,N-1",5"-pentamethyleneacetamidino)-doxorubicin **(57)**,
3'-deamino-3'-(N,N-1",6"-hexamethyleneacetamidino)-doxorubicin hydrochloride **(58)**,
3'-deamino-3'-(N,N-3"-methylaza-1",5"-pentamethyleneacetamidino)-doxorubicin hydrochloride **(59)**,
3'-deamino-3'-(N,N-3"-methylaza-1",5"-pentamethyleneacetamidino)-doxorubicin **(60)**,
3'-deamino-3'-(N,N-o-ethylenephenyleneacetamidino)-doxorubicin hydrochloride **(61)**,
3'-deamino-3'-(N,N-o-ethylenephenyleneacetamidino)-doxorubicin **(62)**,
3'-deamino-3'-(N,N-diethylacetamidino)-doxorubicin hydrochloride **(63)**,
3'-deamino-3'-(N,N-diethylacetamidino)-doxorubicin **(64)**,
3'-deamino-3'-(N,N-dipropylacetamidino)-doxorubicin hydrochloride **(65)**,
3'-deamino-3'-(N,N-dipropylacetamidino)-doxorubicin **(66)**,
3'-deamino-3'-(N,N-dibutylacetamidino)-doxorubicin hydrochloride **(67)**,
3'-deamino-3'-(N,N-dibutylacetamidino)-doxorubicin **(68)**,
3'-deamino-3'-(N,N-1",4"-tetramethyleneformamidino)-epidaunorubicin hydrochloride **(69)**,
3'-deamino-3'-(N,N-1",4"-tetramethyleneformamidino)-epidaunorubicin(70),
3'-deamino-3'-(N,N-3"-oxa-1",5"-pentamethyleneformamidino)-epidaunorubicin hydrochloride **(71)**,
3'-deamino-3'-(N,N-3"-oxa-1",5"-pentamethyleneformamidino)-epidaunorubicin **(72)**,
3'-deamino-3'-(N,N-1",5"-pentamethyleneformamidino)-epidaunorubicin hydrochloride **(73)**,
3'-deamino-3'-(N,N-1",5"-pentamethyleneformamidino)-epidaunorubicin **(74)**,
3'-deamino-3'-(N,N-1",6"-hexamethyleneformamidino)-epidaunorubicin hydrochloride **(75)**.
3'-deamino-3'-(N,N-3'-methylaza-1",5"-pentamethyleneformamidino)-epidaunorubicin hydrochloride **(76)**,
3'-deamino-3'-(N,N-3"-methylaza-1",5"-pentamethyleneformamidino)-epidaunorubicin **(77)**,
3'-deamino-3'-(N,N-o-ethylenephenyleneformamidino)-epidaunorubicin hydrochloride **(78)**,
3'-deamino-3'-(N,N-o-ethylenephenyleneformamidino)-epidaunorubicin **(79)**,
3'-deamino-3'-(N,N-diethylformamidino)-epidaunorubicin hydrochloride **(80)**,
3'-deamino-3'-(N,N-diethylformamidino)-epidaunorubicin **(81)**,
3'-deamino-3'-(N,N-dipropylformamidino)-epidaunorubicin hydrochloride **(82)**,
3'-deamino-3'-(N,N-dipropylformamidino)-epidaunorubicin**(83)**,
3'-deamino-3'-(N,N-dibutylformamidino)-pidaunorubicin hydrochloride **(84)**,
3'-deamino-3'-(N,N-dibutylformamidino)-epidaunorubicin **(85)**,
3'-deamino-3'-[N-methyl-N-(1"-phenylethyl)-formamidino]-epidaunorubicin hydrochloride **(86)**,
3'-deamino-3'-(N,N-1°,4°-tetramethyleneacetamidino)-epidaunonrbicin hydrochloride **(87)**,
3'-deamino-3'-(N,N-1",4"-tetramethyleneacetamidino)-epidaunorubicin**(88)**,
3'-deamino-3'-(N,N-3"-oxa-1",5"-pentamethyleneacetamidino)-epidaunorubicin hydrochloride **(89)**.
3'-deamino-3'-(N,N-3"-oxa-1",5"-pentamethyleneacetamidino)-epidaunorubicin **(90)**,
3'-deamino-3'-(N,N-1",5"-pentamethyleneacetamidino)-epidaunorubicin hydrochloride **(91)**,
3'-deamino-3'-(N,N-1",5"-pentamethyleneacetamidino)-epidaunorubicin **(92)**,
3'-deamino-3'-(N,N-1",6"-hexamethyleneacetamidino)-epidaunorubicin hydrochloride **(93)**,
3'-deamino-3'-(N,N-3"-methylaza-1",5"-pentamethyleneacetamidino)-epidaunorubicin hydrochloride **(94)**,
3'-deamino-3'-(N,N-3"-methylaza-1",5"-pentamethyleneacetamidino)-epidaunorubicin **(95)**,
3'-deamino-3'-(N,N-o-ethylenephenyleneacetamidino)-epidaunorubicin hydrochloride **(96)**,
3'-deamino-3'-(N,N-o-ethylenephenyleneacetamidino)-epidaunorubicin **(97)**,
3'-deamino-3'-(N,N-diethylacetamidino)-epidaunorubicin hydrochloride **(98)**,
3'-deamino-3'-(N,N-diethytacetamidino)-epidaunorubicin **(99)**,
3'-deamino-3'-(N,N-dipropylacetamidino)-epidaunorubicin hydrochloride **(100)**,
3'-deamino-3'-(N,N-dipropylacetamidino)-epidaunorubicin **(101)**,
3'-deamino-3'-(N,N-dibutylacetamidino)-epidaunorubicin hydrochloride **(102)**,
3'-deamino-3'-(N,N-dibutylacetamidino)-epidaunorubicin **(103)**,
3'-deamino-3'-[N-methyl-N-(1"-phenylethyl)-acetamidino]-epidaunorubicin hydrochloride **(104)**,
3'-deamino-3'-(N,N-1",4"-tetramethyleneformamidino)-epidoxorubicin hydrochloride **(105)**,
3'-deamino-3'-(N,N-1",4"-tetramethyleneformamidino)-epidoxorubicin **(106)**,
3'-deamino-3'-(N,N-3"-oxa-1",5"-pentamethyleneformamidino)-epidoxorubicin hydrochloride **(107)**,
3'-deamino-3'-(N,N-3"-oxa-1",5"-pentamethyleneformamidino)-epidoxorubian **(108)**,
3'-deamino-3'-(N,N-1",5"-pentamethyleneformamidino)-epidoxorubicin hydrochloride **(109)**,
3'-deamino-3'-(N,N-1",5"-pentamethyleneformamidino)-epidoxorubicin **(110)**,
3'-deamino-3'-(N,N-1",6"-hexamethyleneformamidino)-epidoxorubicin hydrochloride **(111)**,
3'-deamino-3'-(N,N-3"-methylaza-1",5"-pentamethyleneformamidino)-epidoxorubicin hydrochloride **(112)**,
3'-deamino-3'-(N,N-3"-methylaza-1",5"-pentamethyleneformamidino)-epidoxorubicin **(113)**,
3'-deamino-3'-(N,N-o-ethylenephenyleneformamidino)-epidoxorubicin hydrochloride **(114)**,
3'-deamino-3'-(N,N-o-ethylenephenyleneformamidino)-epidoxorubicin **(115)**,
3'-deamino-3'-(N,N-diethylformamidino)-epidoxorubicin hydrochloride **(116)**,
3'-deamino-3'-(N,N-diethylformamidino)-epidoxonrbicin **(117)**,
3'-deamino-3'-(N,N-dipropylformamidino)-epidoxorubicin hydrochloride **(118)**,
3'-deamino-3'-(N,N-dipropylformamidino)-epidoxorubicin **(119)**,
3'-deamino-3'-(N,N-dibutylformamidino)-epidoxorubicin hydrochloride **(120)**,
3'-deamino-3'-(N,N-dibutylformamidino)-epidoxorubicin **(121)**,
3'-deamino-3'-[N-methyl-N-(1"-phenylethyl)-formamidino]-epidoxorubicin hydrochloride **(122)**,
3'-deamino-3'-(N,N-1",4"-tetramethyleneacetamidino)-epidoxorubicin hydrochloride **(123)**,
3'-deamino-3'-(N,N-1",4"-tetramethyleneacetamidino)-epidoxorubicin **(124)**,
3'-deamino-3'-(N,N-3"-oxa-1",5"-pentamethyleneacetamidino)-epidoxorubicin hydrochloride **(125)**,
3'-deamino-3'-(N,N-3"-oxa-1",5"-pentamethyleneacetamidino)-epidoxorubicin **(126)**,
3'-deamino-3'-(N,N-1",5"-pentamethyleneacetamidino)-epidoxorubicin hydrochloride **(127)**,
3'-deamino-3'-(N,N-1",5"-pentamethyleneacetamidino)-epidoxonibicin **(128)**,
3'-deamino-3'-(N,N-1",6"-hexamethyleneacetamidino)-epidoxorubicin hydrochloride **(129)**,
3'-deamino-3'-(N,N-1",7"-heptamethyleneacetamidino)-epidoxorubicin hydrochloride **(130)**,
3'-deamino-3'-(N,N-3"-methylaza-1",5"-pentamethyleneacetamidino)-epidoxorubicin hydrochloride **(131)**,
3'-deamino-3'-(N,N-3"-methylaza-1",5"-pentamethyleneacetamidino)-epidoxorubicin **(132)**,
3'-deamino-3'-(N,N-o-ethylenephenyleneacetamidino)-epidoxorubicin hydrochloride **(133)**,
3'-deamino-3'-(N,N-o-ethylenephenyleneacetamidino)-epidoxorubicin **(134)**,
3'-deamino-3'-(N,N-diethylacetamidino)-epidoxorubicin hydrochloride **(135)**,
3'-deamino-3'-(N,N-diethylacetamidino)-epidoxorubicin **(136)**,
3'-deamino-3'-(N,N-dipropylacetamidino)-epidoxorubicin hydrochloride **(137)**,
3'-deamino-3'-(N,N-dipropylacetamidino)-epidoxorubicin **(138)**,
3'-deamino-3'-(N,N-dibutylacetamidino)-epidoxorubicin hydrochloride **(139)**,
3'-deamino-3'-(N,N-dibutylacetamidino)-pidoxorubicin **(140)**,
3'-deamino-3'-[N-methyl-N-(1"-phenylethyl)-acetamidino]-pidoxorubicin hydrochloride **(141)**,

Moreover, the following derivatives of the general formula 2, wherein R¹, R² and R⁴ have the above-mentioned meaning, were used for studies of antihelicase activity:
3'-deamino-3'-N, 4'-O-methylidenedaunorubicin **(142)**,
3'-deamino-3'-N, 4'-O-methylidenedoxorubicin **(143)**,
3'-deamino-3'-N, 4'-O-ethylidenedaunorubicin **(144)**,
3'-deamino-3'-N, 4'-O-ethylidenedoxorubicin **(145)**.

The studies were carried out with the use of a method developed by the Inventors (Boguszewska-Chachulska A.M. et al., FEBS Lett., 567, 253-238, 2004). Advantages of the method as compared to up-to-now used isotope methods (Kim D.W. et al., Biochemistry Biophysics Research Communications 215, 160-166, 1995; and Borowski P. et al, European J. Biochemistry 270, 1645-1653, 1999) are the following: safety, repeatability and possibility to test large amount of samples, e.g. of various inhibitors of helicase at various concentrations, in a number of repetitions, in standard conditions and during a very short time. A reaction takes place on a 96- or 384-well microtitration plate and a course of the reaction is observed in real time.

The above-described method was used to study an antihelicase activity of amidinoanthracyclines of general formula 1 or 2, wherein R¹, R², R³, R⁴, R⁵, R⁶ and X have the meaning defined above, and - for comparison - of the activity of the parent antibiotics, such as daunorubicin, doxorubicin, epidaunorubicin and epidoxorubicin. The results obtained using the above-mentioned method indicate, that the studied amidinoanthracyclines of general formula 1, wherein R¹, R², R³, R⁴. R⁵, R⁶ and X have the above-given meaning, exhibited potent antihelicase action (the IC₅₀ values in the range of 0.03-11.32 µM) and a distinct dependence of the activity on the structure of the studied amidino derivatives. It appeared that the most active acetamidino derivatives were those of epidaunorubicin and epidoxorubicin (Table 1), the IC₅₀ values of which were in the range of 0.03-0.99 µM. In the case of formamidino derivatives the most active (the IC₅₀ values in the range of 0.36-1.84 µM) were derivatives of the above-mentioned parent antibiotics having pyrrolidine moiety in the amidino group (Table 1 and 2), whereas the least active (the IC₅₀ values ranging from 9.26 to ≥10 µM) were derivatives of general formula 2, wherein R¹, R and R⁴ have the above-given meaning, containing modified daunosamine moiety (compounds 142-145).

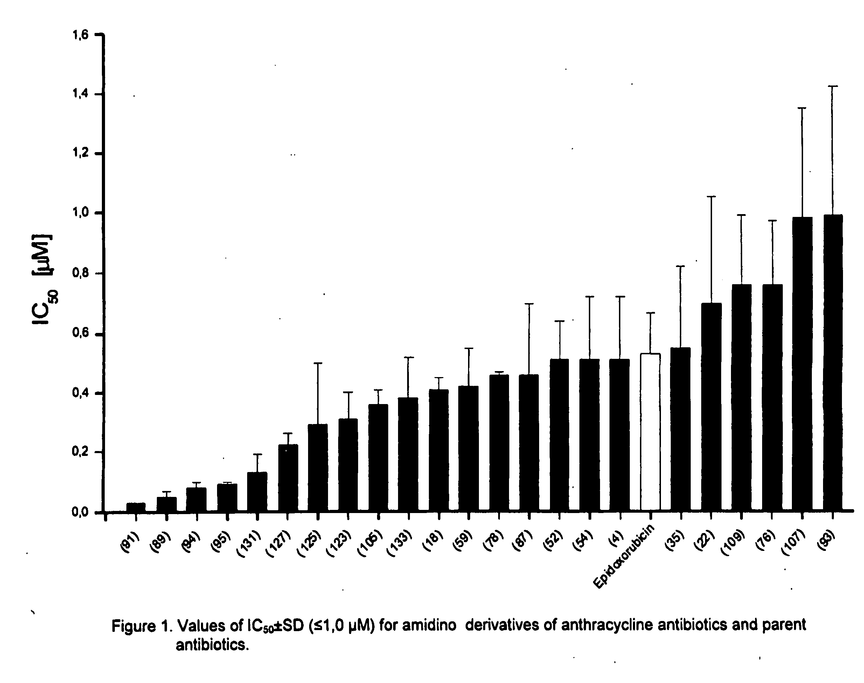

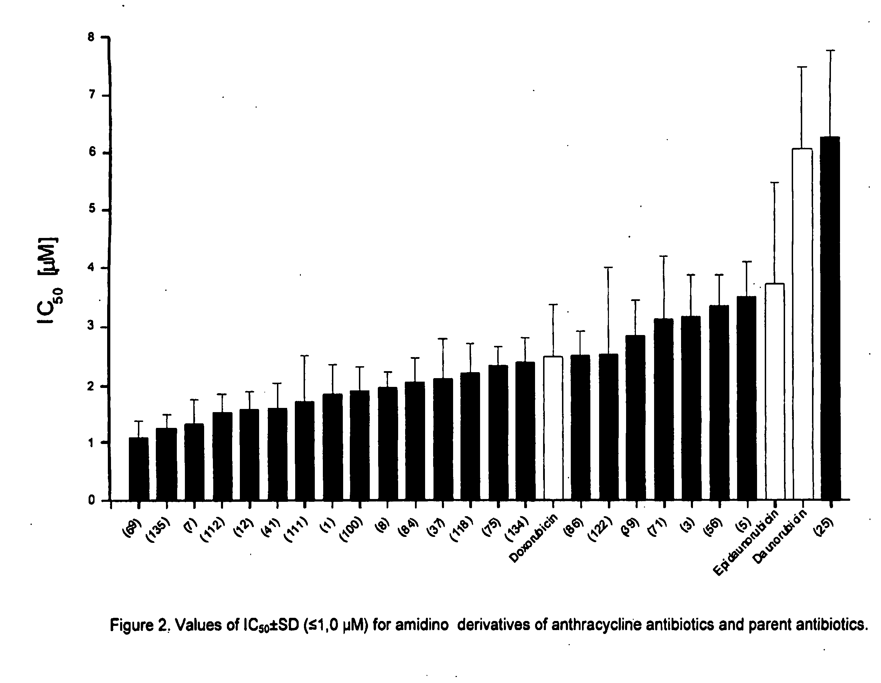

The most values of the IC₅₀ given in Table 1 and 2 and illustrated by Figures 1 and 2 pertain to hydrochlorides of derivatives of the formula 1, wherein R¹, R², R³, R⁴, R⁵, R⁶ have the meaning defined above, and X is HCl. Studies of activity of these compounds as free bases (if X is 0) exhibited slight differences in the IC₅₀ values between hydrochloride and the corresponding free base. E.g., for hydrochloride of 3'-deamino-3'-(N,N-3"-methylaza-1",5"-pentamethyleneformamidino)-epidaunorubicin (76) and for corresponding free base - 3'-deamino-3'-(N,N-3"-**methylaza-1",5"-pentamethyleneformamidino)-epidaunorubicin (77)** these values were: 0.76 ± 0.21 µM and 0.78 ± 0.18 µM, respectively, whereas for hydrochloride of 3'-deamino-3'-(N,N-3"-methylaza-1",5"-pentamethyleneacetamidino)-epidaunorubi**cin(94)** and the free base - 3'-deamino-3'-(N,N-3"-methylaza-1",5"-pentamethyleneacetamidino)-epidaunorubicin **(95)** were 0.08 ± 0.02 and 0.09 ± 0.01 µM, respectively. Because of that it was assumed that the IC₅₀ values for free bases correspond to the IC₅₀ values for hydrochlorides of compounds of general formulae 1 or 2, within IC₅₀ ± 10%.

Therefore the IC₅₀ values for formamidinoanthracyclines as free bases, hydrochlorides of which are listed in Table 1, such as: 3'-deamino-3'-(N,N-1",4'-tetramethyleneformamidino)-doxorubicin **(36)**, 3'-deamino-3'-(N,N-3"-methylaza-1",5"-pentamethyleneformamidino)-doxorubicin **(43)**, 3'-deamino-3'-(N,N-3"-methylaza-1",5"-pentamethyleneformamidino)-epidaunorubicin **(77)**, 3'-deamino-3'-(N,N-o-ethylenephenyleneformamidino)-epidaunorubicin **(79)**, 3'-deamino-3'-(N,N-diethylformamidino)-epidaunorubicin **(81)**, 3'-deamino-3'-(N,N-1",4"-tetramethy)eneformamidino)-epidoxorubicin(106), 3'-deamino-3'-(N,N-3"-oxa-1",5"-pentamethyleneformamidino)-epidoxorubicin **(108)**, 3'-deamino-3'-(N,N-1",5" -pentamethyleneformamidino)-epidoxorubicin **(110)** and 3'-deamino-3'-(N,N-o-ethylenephenyleneformamidino)-epidoxorubicin **(115)** are within the limits of 0.4-1.2 µM, whereas analogolously for acetamidinoanthracyclines as free bases (Table 1), such as: 3'-deamino-3'-(N,N-1",4"-tetramethyleneacetamidino)-daunorubicin **(19)**, 3'-deamino-3'-(N,N-1",5"-pentamethyleneacetamidino)-daunorubicin **(23)**, 3'-deamino-3'-(N,N-1",4"-tetramethyleneacetamidino)-doxorubicin **(53)**, 3'-deamino-3'-(N,N-3"-oxa-1",5"-pentamethyleneacetamidino)-doxorubicin **(55)**, 3'-deamino-3'-(N,N-3"-methylaza-1",5"-pentamethyleneacetamidino)-doxorubicin **(60)**, 3'-deamino-3'-(N,N-1",4"-tetramethyleneacetamidino)-epidaunorubicin **(88)**, 3'-deamino-3'-(N,N-3"-oxa-1",5"-penta-methyleneacetamidino)-epidaunorubicin **(90)**, 3'-deamino-3'-(N,N-1",5"-pentamethyleneacetamidino)-epidaunorubicin **(92)**, 3'-deamino-3'-(N,N-diethylacetamidino)-epidaunorubicin **(99)**, 3'-deamino-3'-(N,N-1",4"-tetramethyleneacetamidino)-epidoxorubicin . **(124)**, 3'-deamino-3'-(N,N-3"-oxa-1",5"-pentamethyleneacetamidino)-epidoxorubicin **(126)**, 3'-deamino-3'-(N,N-1",5"-pentamethyleneacetamidino)-epidoxorubicin **(128)**, 3'-deamino-3'-(N,N-3"-methylaza-1",5"-pentamethyleneacetamidino)-epidoxorubicin **(132)** and 3'-deamino-3'-(N,N-o-ethylenephenyleneacetamidino)-epidoxorubicin **(134)**, within the range 0.08-1.1 µM, respectively.

In Table 2 antihelicase activity (≥1.0 µM) of derivatives of general formula 1, wherein R¹, R², R³, R⁴, R⁵, R⁶ and X have the meaning defined above, is presented. That group of compounds includes mainly formamidinoanthracyclines of activity within the IC₅₀ values ranging from 1.08 to 3.5 µM and few acetamidinoanthracyclines of the IC₅₀ values in the range of 1.24-11.32 µM.

Antihelicase activity of free bases corresponding to hydrochlorides of formamidinoanthracyclines listed in Table 2, such as 3'-deamino-3'-(N,N-1",4"-tetramethyleneformamidino)-daunorubicin **(2)**, 3'-deamino-3'-(N,N-3"-oxa-1",5"-pentamethyleneformamidino)-daunorubicin **(4)**, 3'-deamino-3'-(N,N-1",5"-pentamethyleneformamidino)-daunorubicin **(6)**, 3'-deamino-3'-(N,N-3"-methylaza-1",5"-pentamethyleneformamidino)-daunorubicin **(9)**, 3'-deamino-3'-(N,N-diethylformamidino)-daunorubicin **(13)**, 3'-deamino-3'-(N,N-3"-oxa-1",5"-pentamethyleneformamidino)-doxorubicin **(38)**, 3'-deamino-3'-(N,N-1",5"-pentamethyleneformamidino)-doxorubicin **(40)**, 3'-deamino-3'-(N,N-diethylformamidino)-doxorubicin **(47)**, 3'-deamino-3'-(N,N-1",4"-tetramethyleneformamidino)-epidaunorubicin **(70)**, 3'-deamino-3'-(N,N-3"-oxa-1",5"-pentamethyleneformamidino)-epidaunorubicin **(72)**, 3'-deamino-3'-(N,N-1",5"-pentamethyleneformamidino)-epidaunorubicin **(74)**, 3'-deamino-3'-(N,N-dibutylformamidino)-epidaunorubicin **(85)**, 3'-deamino-3'-(N,N-3"-methylaza-1",5"-pentamethyleneformamidino)-epidoxorubicin **(113)** 3'-deamino-3'-(N,N-diethylformamidino)-epidoxorubicin **(117)** and 3'-deamino-3'-(N,N-dipropylformamidino)-epidoxorubicin **(119)**, expressed as the IC₅₀ values, were in the range of 1.2-3.9 µM, whereas activity of free bases -acetamidinoanthracyclines (Table 2), such as 3'-deamino-3'-(N,N-3"-methylaza-1",5"-pentamethyleneacetamidino)-daunorubicin **(26)**, 3'-deamino-3'-(N,N-o-ethylenephenyleneacetamidino)-daunorubicin **(28)**, 3'-deamino-3'-(N,N-1",5"-pentamethyleneacetamidino)-doxorubicin **(57)**, 3'-deamino-3'-(N,N-dipropylacetamidino)-epidaunorubicin **(101)** and 3'-deamino-3'-(N,N-diethylacetamidino)-epidoxorubicin **(136)**, expressed as the IC₅₀ values were in the range of 7.0-13.0 µM.

It is important that compounds not mentioned above of general formula 1, wherein R¹, R², R⁵, R⁴, R⁶, R⁶ and X have the meaning defined above, such as 3'-deamino-3'-(N,N-o-ethylenephenyleneformamidino)-daunorubicin hydrochloride **(10)**, 3'-deamino-3'-(N,N-o-ethylenephenyleneformamidino)-daunorubicin **(11)**, 3'-deamino-3'-(N,N-dipropylformamidino)-daunorubicin hydrochloride **(14)**, 3'-deamino-3'-(N,N-dipropylformamidino)-daunorubicin **(15)**, 3'-deamino-3'-(N,N-dibutylformamidino)-daunorubicin hydrochloride **(16)**, 3'-deamino-3'-(N,N-dibutylformamidino)-daunorubicin **(17)**, 3'-deamino-3'-(N,N-3"-oxa-1",5"-pentamethyleneacetamidino)-daunorubicin hydrochloride **(20)**, 3'-deamino-3'-(N,N-3"-oxa-1",5"-pentamethyleneacetamidino)-daunorubicin **(21)**, 3'-deamino-3'-(N,N-1",6"-hexamethyleneacetamidino)-daunorubicin hydrochloride **(24)**, 3'-deamino-3'-(N,N-diethylacetamidino)-daunorubicin hydrochloride **(29)**, 3'-deamino-3'-(N,N-diethylacetamidino)-daunorubicin **(30)**, 3'-deamino-3'-(N,N-dipropylacetamidino)-daunorubicin hydrochloride **(31)**, 3'-deamino-3'-(N,N-dipropylacetamidino)-daunorubicin **(32)**, 3'-deamino-3'-(N,N-dibutylacetamidino)-daunorubicin hydrochloride **(33)**, 3'-deamino-3'-(N,N-dibutylacetamidino)-daunorubicin **(34)**, 3'-deamino-3'-(N,N-o-ethylenephenyleneformamidino)-doxorubicin hydrochloride, **(44)**, 3'-deamino-3'-(N,N-o-ethylenephenyleneformamidino)-doxorubicin **(45)**, 3'-deamino-3'-(N,N-dipropylformamidino)-doxorubicin hydrochloride **(48)**, 3'-deamino-3'-(N,N-dipropylformamidino)-doxorubicin **(49)**, 3'-deamino-3'-(N,N-dibutylformamidino)-doxorubicin hydrochloride **(50)**, 3'-deamino-3'-(N,N-dibutylformamidino)-doxorubicin **(51)**,3'-deamino-3'-(N,N-1",6"-hexametyleneacetamidino)-doxorubicin hydrochloride **(58)**, 3'-deamino-3'-(N,N-o-ethylenephenyleneacetamidino)-doxorubicin hydrochloride(61),3'-deamino-3'-(N,N-o-ethylenephenyleneacetamidino)-doxorubicin **(62)**, 3'-deamino-3'-(N,N-diethylacetamidino)-doxorubicin hydrochloride **(63)**, 3'-deamino-3'-(N,N-diethylacetamidino)-doxorubicin **(64)**, 3'-deamino-3'-(N,N-dipropylacetamidino)-doxorubicin hydrochloride **(65)**, 3'-deamino-3'-(N,N-dipropylacetamidino)-doxorubicin **(66)**, 3'-deamino-3'-(N,N-dibutylacetamidino)-doxorubicin hydrochloride **(67)**, 3'-deamino-3'-(N,N-dibutylacetamidino)-doxorubicin **(68)**, 3'-deamino-3'-(N,N-dipropylformamidino)-epidaunorubicin hydrochloride **(82)**, 3'-deamino-3'-(N,N-dipropylformamidino)-epidaunorubicin **(83)**, 3' -deamino-3' -(N,N-o-ethylenephenyleneacetamidino)-epidaunorubicin hydrochloride **(96)**, 3'-deamino-3'-(N,N-o-ethylenephenyleneacetamidino)-epidaunorubicin **(97)**, 3'-deamino-3'-(N,N-dibutylacetamidino)-epidaunorubicin hydrochloride **(102)**, 3'-deamino-3'-(N,N-dibutylacetamidino)-epidaunorubicin **(103)**, 3'-deamino-3'-[N-methyl-N-(1"-phenylethyl)-acetamidino]-epidaunorubicin hydrochloride **(104)**, 3'-deamino-3'-(N,N-dibutylformamidino)-epidoxorubicin hydrochloride **(120)**, 3'-deamino-3'-(N,N-dibutylformamidino)-epidoxorubicin **(121)**, 3'-deamino-3'-(N,N-1",6"-hexamethyleneacetamidino)-epidoxorubicin hydrochloride **(129)**, 3'-deamino-3'-(N,N-1",7"-heptamethyleneacetamidino)-epidoxorubicin hydrochloride **(130)**, 3'-deamino-3'-(N,N-dipropylacetamidino)-epidoxorubicin hydrochloride **(137)**, 3'-deamino-3'-(N,N-dipropylacetamidino)-epidoxorubicin **(138)**, 3'-deamino-3'-(N,N-dibutylacetamidino)-epidoxorubicin hydrochloride **(139)**, 3'-deamino-3'-(N,N-dibutylacetamidino)-epidoxorubicin **(140)** and 3'-deamino-3'-[N-methyl-N-(1"- phenylethyl)-acetamidino]-epidoxorubicin hydrochloride **(141)** also exhibit potent antihelicase action. The IC₅₀ values of the above-mentioned derivatives are in the range of 0.1-10.0 µM.

It should be pointed out that the most potent antihelicase action exhibited these amidinoanthracyclines which in the group of investigated derivatives of the formulae 1 or 2, wherein R¹, R², R³, R⁴, R⁵, R⁶ and X have the meaning defined above, had the weakest antineoplastic action, which, as it is known, is advantageous when studying antiviral action of individual compounds.

Idarubicine, an orally administered analogue of daunorubicin, having hydrogen in position 3 and its amidinoderivatives - prepared by the Inventors - as 3'-deamino-3'-(N,N-3"-oxa-1",5"-pentamethyleneformamidino)-idarubicin hydrochloride of the formula 1, wherein R¹, R² and R⁴ are hydrogen, R³ is hydroxy group in an axial orientation, whereas R⁵ and R⁶ together with a nitrogen atom are N,N-3"-oxa-1",5"-pentamethylene group and 3'-deamino-3'-(N,N-1",6"-hexamethyleneformamidino)-idarubicin hydrochloride of formula 1, wherein R¹, R² and R⁴ are hydrogen, R³ is hydroxy group in an axial orientation, whereas R⁵ and R⁶ together with a nitrogen atom form N,N-1",6"-hexamethylene group, also exhibited an antihelicase action, but the action was less potent (≥10 µM) than that of the parent anthracycline antibiotics discussed herein, as well as that of their derivatives of general formula 1.

As it has been found in several studies, an antihelicase activity of compounds is mainly associated with their antiviral activity. This results from the crucial role of NS3 protein in the replication cycle of *Flaviviridae* viruses, which because of that can be a target of antiviral chemotherapy (Tan S-L at al., Nature Reviews/ Drug Discovery 1, 867-881, 2002, Gordon C.P. and Keller P.A., J. Med. Chem. 48, 1-20, 2005). For example, a study concerning inhibition of multiplication of a virus of the *Flaviviridae* family (Western Nile Virus, WNV) conducted by Borowski and coil. (Borowski P. et al., Antimicrobial Agent and Chemotherapy 46, 1231-1239, 2002, and Borowski P. et al, (Acta. Biochim. Polon. 49, 597-614, 2002) proves that a nucleoside analogue, which is an inhibitor of viral helicase activity under conditions without an incubation with a substrate, at the same time is an effective inhibitor of virus replication in a cell line, which in both cases makes it possible to attain 50% inhibition at similar concentration (approx. 30 µM). Studies of inhibitors of helicase-primase of herpes simplex virus (HSV), using modified nucleosides analogues, also exhibited that these compounds can be effective inhibitors of viral replication, 50% inhibition of replication occurring at lower concentrations than inhibition of helicase activity (Crute J.J. et al., Nature Medicine 8, 386-391, 2002).

The derivatives in question of general formulae 1 or 2 are subject to further testing in HCV-infected human lymphocytes and peripheral blood mononuclear cells (PBMC).

Studies on inhibition of HCV replication, using human liver cancer cell line (Huh7) carrying a subgenomic HCV replicon of genotype 1 (con1) with a reporter/selection fusion protein luc-ubi-neo, were also carried out. Results of determination of antiviral activity and therapeutic index of selected amidinoanthracyclines are given in Table 3. It has been found that the derivatives in question reduced level of HCV RNA in a concentration-dependent manner, efficiently inhibiting replication of HCV at nanomolar concentrations (from 8 to 140 nM) without cell cytotoxicity, with exception of an epidaunorubicin derivative (75), which, however, is the most potent inhibitor, presenting activity (EC₅₀ = 8.4 nM) 4 fold higher than that of the parent antibiotic (EC₅₀ = 31.18 nM).

Although a doxorubicin derivative (54), has higher EC₅₀ than the parent doxorubicin, it exhibits the lowest cytotoxicity of the studied compounds and the highest therapeutic index. It should be pointed out that all derivatives that were compared exhibited high therapeutic indices (5.7, 8.0, 11.4, 19.2 and 33.3), whereas for ribavirin - a drug used at present in treatment of infection by HCV - the index was merely 1.5, at EC₅₀ = 120 µM. These data suggest that derivatives (54, 75, 94, 125, 133) can be more preferable components of future multicomponent therapy against HCV than ribavirin.

According to the invention, the medical use of derivatives of general formulae 1 or 2, wherein R¹, R², R³, R⁴, R⁵, R⁶ and X have the meaning defined above, consists in that the derivatives, inhibitors of NS3 helicase of hepatitis C virus (HCV), are used for preparation of a medicine which inhibits the helicase.

The medical use of derivatives of general formulae 1 and 2, wherein R¹, R², R³, R⁴, R⁵, R⁶ and X have the above-given meaning, consists in that the derivatives are used for preparation of a medicine for treatment of infection caused by hepatitis C virus (HCV), a dosage of the medicine being administered in such a way that it causes a constant inhibition of viremia. This dose is 0,005-1,5 mg/kg corresponding to 0,25-0,75 mg/m².

An additional aspect of the invention is a pharmaceutical composition comprising pharmaceutically acceptable carriers, diluents, rexcipients and anthracycline antibiotic/s of general formula 1 or 2, as active ingredient, wherein R¹, is hydrogen or methoxy group. R² is hydrogen or hydroxy group, R³ is hydroxy group in an axial or equatorial orientation, R⁴ and R⁵ are hydrogen or methyl group, R⁶ is 1-phenylethyl group or R⁵ and R⁶ together with a nitrogen atom are N,N-diethyl, N,N-dipropyl or N,N-dibutyl group, or R⁵ and R⁶ together with a nitrogen atom form N,N-1",4"-tetramethylene, N,N-3"-oxa-1",5"-penta-methylene, N,N-1",5"-pentamethylene, N,N-1",6"-hexamethylene, N,N-1",7"-heptamethylene, N,N-3"-methylaza-1",5"-pentamethylene or 1-indolinyl group, whereas if X is HCl molecule, the compound is hydrochloride of a derivative of general formula 1 or if X is 0, the compound is a derivative in a free base form of general formula 1 for use in the treatment of infection caused by hepatitis C virus.

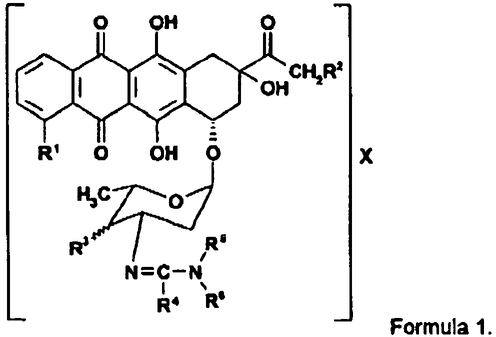

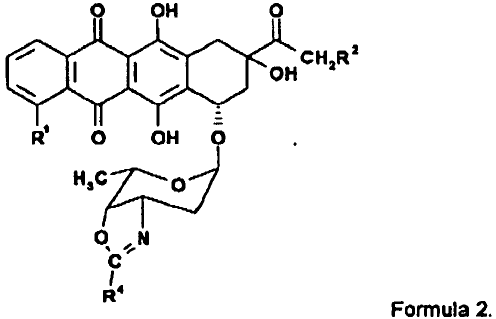

The pharmaceutical composition is prepared by a method of pharmaceutical form preparation known from the art.

According to the invention pharmaceutical compositions exhibiting antihelicase activity comprising known pharmaceutically acceptable carriers and diluents, are characterised by the fact that as an active ingredient(s) they include a derivative or derivatives of anthracycline antibiotic of general formula 1 or 2, wherein R¹, R², R³, R⁴, R⁵, R⁶ and X have the above-given meaning or derivative or derivatives of general formula 1 or 2, combined with the parent anthracycline antibiotic or also derivative or derivatives of general formula 1 or 2, combined with another antiviral drug.

As pharmaceutically acceptable carriers and diluents preferably lactose, nipagin, dextrose, glucose or 0.9% sodium chloride solution are used.

Derivatives according to the invention, of general formula 1 or 2, can be administered as injections or infusions. For the purpose of parenteral administration, unit dosage forms are prepared as solutions that include each of the compounds according to the invention and an aseptic diluent. When preparing the solution, the active ingredient can be dissolved in a solution for injections and sterilized by filtering. The resulting sterile solution is used to fill vials or ampoules, which are then closed under aseptic conditions. The solution in vials can also be lyophilized to obtain formulation as a dry powder. In such cases a second vial is added to a tyophilizate including water or solution for injections, to prepare an injection drug form. The above-mentioned drug forms as an active ingredient can also include free bases or hydrochlorides of each of the compounds according to the invention.

Accordingly, derivatives of general formulae 1 or 2, wherein R¹, R², R³, R⁴, R⁵, R⁶ and X have the above-given meaning, exhibit potent antihelicase action and - previously disclosed-considerably lower toxicity as compared to the parent anthracycline antibiotics, and can be preferably used for treatment of HCV infections in the future.

The examples illustrate the invention.

### Legend to the Figures:

- Figure 1.: Values of IC₅₀±SD (≤1,0 µM) present activity for amidino derivatives of anthracycline antibiotics and parent antibiotics.
- Figure 2.: Values of IC₅₀±SD (≤1,0 µM) shows activity for amidino derivatives of anthracycline antibiotics and parent antibiotics.

### Example 1. The examination of antihelicase activity.

We expressed the helicase domain of NS3 protein in a baculovirus system in HF insect cell line. The helicase protein obtained in the baculovirus system was purified using a method developed by us (Boguszewska-Chachulska A.M. et al., FEBS Lett. 567: 253-258, 2004) and consists of following steps: stage I - chromatographic separation of insect cells lysate on a Talon column (Clontech), using affinity of cobalt ions to histidine repetitions, and elution of the protein from the column with 250 mM imidazole; stage II - further purification of the protein by affinity method on a heparin column HiTrap (Amersham Biosciences) using Biologic (BioRad) or AKTA (Amersham Biosciences) system, optionally followed by concentration of the eluate with Amicon Ultra filters (Millipore).

We established (Boguszewska-Chachulska A.M. et al., FEBS Lett. 567: 253-258, 2004) a fluorometric assay based on the use of a double-stranded DNA substrate where one strand was labelled at the 5'end with a fluorophore (FAM) and the second was labelled at 3'end with a molecule absorbing fluorescence - Black Hole Quencher (BHQ1). FAM fluorescence is quenched by fluorescence energy transfer (FRET) to the BHQ molecule, while unwinding of double-stranded DNA structure leads to decrease of FRET phenomenon and increase of FAM fluorescence signal. The assay allows to examine a big number of samples, i.e. different helicase inhibitors at various concentrations and with many repetitions, in standardized conditions. The reaction is conducted in a microtitration plate. The reaction process is observed in real time.

The examination of potential inhibitors of HCV helicase was conducted in conditions based on a work of Boguszewska-Chachulska A.M. et al. (Biochem Biophys Res Commun. 34: 641-647, 2006). Helicase reactions were performed in the following mixture: 30 mM Tris-HCl, pH 7.5, 10 mM MnCl₂, 0.075% Triton X-100, 0.05% sodium azide, 10 nM substrate, 1.5 mM ATP, 125 nM of oligonucleotide complementary to BHQ1-labelled strand in the 60 µl reaction volume. The enzyme (10 nM) was pre-incubated with the tested derivatives dissolved in DMSO (0.005 to 20 µM concentration) without ATP for 15 min. at room temperature. The reaction of DNA unwinding was started by addition of ATP and was carried out at 30°C for 60 min. in a Synergy HTi (Biotek) fluorometer. Since the excitation maximum for FAM is at 495 nm, and the emission maximum is at 520 nm, the fluorophore was excited at 485/20 nm filter, and the helicase activity was measured at 528/20 nm. Fluorescence was measured every 2 mins.

The enzyme activity was calculated as the initial reaction velocity from the linear part of reaction curve (fluorescence growth as a function of time) using the linear regression method. Inhibition was calculated as comparison of activity of the helicase incubated with inhibitor to activitly of the helicase incubated without inhibitor, but with a proper concentration of DMSO, expressed in percents. Obtained IC₅₀ values in µM (molar concentration of examined compound which inhibits 50% of helicase activity) are given in Tables 1 and 2.

Additionally, Figure 1 presents activity of derivatives and the parent compound showing values of IC₅₀ ≤1 µM, while Figure 2 shows activity of derivatives and the parent compounds showing values of IC₅₀ >1.0 µM.

**Table 1. IC₅₀±SD (≤ 1.0µM) values for amidino derivatives of anthracycline antibiotics and the parent antibiotics**

| Number of derivative | IC₅₀ [µM] | SD [µM] |
|---|---|---|
| **(91)** | 0.03 | 0.00 |
| **(89)** | 0.05 | 0.02 |
| **(94)** | 0.08 | 0.02 |
| **(95)** | 0.09 | 0.01 |
| **(131)** | 0.13 | 0.06 |
| **(114)** | 0.14 | 0.02 |
| **(98)** | 0.18 | 0.02 |
| **(127)** | 0.22 | 0.04 |
| **(125)** | 0.29 | 0.21 |
| **(80)** | 0.30 | 0.07 |
| **(123)** | 0.31 | 0.09 |
| **(105)** | 0.36 | 0.05 |
| **(133)** | 0.38 | 0.14 |
| **(18)** | 0.41 | 0.04 |
| **(59)** | 0.42 | 0.13 |
| **(78)** | 0.45 | 0.01 |
| **(87)** | 0.46 | 0.24 |
| **(52)** | 0.50 | 0.13 |
| **(54)** | 0.51 | 0.21 |
| **Epidoxorubicin** | 0.53 | 0.14 |
| **(35)** | 0.55 | 0.27 |
| **(22)** | 0.70 | 0.35 |
| **(109)** | 0.76 | 0.23 |
| **(76)** | 0.77 | 0.21 |
| **(107)** | 0.97 | 0.07 |
| **(42)** | 0.98 | 0.11 |
| **(93)** | 0.99 | 0.86 |

| | | |
|---|---|---|
| **^{*)} sorted by ascending IC₅₀ values.** | | |

**Table 2. IC₅₀±SD (> 1.0µM) values for amidino derivatives of anthracycline antibiotics and the parent antibiotics^{*)}**

| Number of derivative | IC₅₀ µM | SD µM |
|---|---|---|
| **(69)** | 1.08 | 0.30 |
| **(135)** | 1.24 | 0.26 |
| **(46)** | 1.32 | 0.07 |
| **(7)** | 1.33 | 0.43 |
| **(112)** | 1.53 | 0.31 |
| **(12)** | 1.58 | 0.30 |
| **(41)** | 1.61 | 0.42 |
| **(111)** | 1.72 | 0.78 |
| **(1)** | 1.84 | 0.52 |
| **(100)** | 1.90 | 0.42 |
| **(8)** | 1.96 | 0.26 |
| **(84)** | 2.05 | 0.41 |
| **(37)** | 2.12 | 0.66 |
| **(116)** | 2.17 | 0.45 |
| **(118)** | 2.21 | 0.51 |
| **(75)** | 2.33 | 0.33 |
| **(73)** | 2.39 | 0.41 |
| **Doxorubicin** | 2.48 | 0.88 |
| **(86)** | 2.50 | 0.42 |
| **(122)** | 2.53 | 1.47 |
| **(39)** | 2.84 | 0.61 |
| **(71)** | 3.13 | 1.06 |
| **(3)** | 3.17 | 0.71 |
| **(56)** | 3.35 | 0.52 |
| **(5)** | 3.50 | 0.59 |
| **Epidaunorubicin** | 3.73 | 1.73 |
| **Daunorubicin** | 6.05 | 1.43 |
| **(25)** | 6.25 | 1.51 |
| **(142)** | 9.26 | 2.03 |
| **(27)** | 11.32 | 0.98 |
| **(144)** | >6 | - |
| **(143)** | >10 | - |
| **(145)** | >10 | - |

| | | |
|---|---|---|
| ^{*)} sorted by ascending IC₅₀ values. | | |

### Example 2. The examination of antiviral activity and determination of therapeutic index

In order to examine antiviral activity and to determine therapeutic index of selected derivatives from the amidinoanthracycline group we used a subgenomic replicon of hepatitis C virus, which is a modified genome of HCV lacking structural genes. The replicon is a non-infective form of the virus, and therefore is safe to work with and makes it easier high-throughput screening of potential inhibitors of its replication.

Additional simplification is the use of reporter protein - luciferase, whose activity (i.e. luminescence generated by it after addition of substrate) is proportional to the amount of the protein expressed, which allows determination of HCV RNA replication level and its inhibition by virus replication inhibitors.

The human hepatoma cell line Huh-7 and the plasmid pFK-luc-ubi-neo/NS3-3'/Con1/5.1 carrying the subgenomic HCV genotype 1 (con 1) replicon with the luc-ubi-neo reporter/selective fusion protein (Vrolijk J.M. et al., J. Virol. Methods 110: 201-209, 2003), used in the experiments, were provided by dr Ralf Bartenschlager (Department of Molecular Virology, University of Heidelberg, Heidelberg). Huh-7 clones carrying persistently replicating subgenomic HCV replicons were obtained using a modification of protocol described by Lohman V. et al. (J. Virol. 75:1437-1449, 2001).

Huh-7 cells were grown in minimal essential medium (Dulbecco's Modified Minimal Essential Medium, DMEM; Invitrogen) with high glucose concentration (4.5 g/l) supplemented with 2 mM L-glutamine and 1 µM non-essential amino acids such as: glycine, L-alanine, L-asparagine, L-aspartic acid, L-glutamic acid, L-proline and L-serine, and also with 100 U/ml penicilin, 100 µg/ml streptomycin and 10% fetal bovine serum (FBS, Sigma). In cultures of cells bearing the subgenomic HCV replicon 250 µg/ml of aminoglycoside antibiotics G418 (Geneticin; Invitrogen) was added to the medium. Cells were grown at 37°C (5% CO₂) and were passaged twice per week, diluted 7 to 10 times, according to the protocol (Vrolijk J.M. et al., J. Virol. Methods 110: 201-209, 2003).

The Huh-7 cells carrying replicons were cultivated with the following concentrations of derivatives of anthracycline antibiotics: 5 to 200 nM for 4 days to determine their influence on HCV RNA replication and 5 to 5000 nM for 3 days to determine cytotoxicity. The medium with 1% DMSO was used as a control.

Conditions of the antiviral activity test of antracycline derivatives were based on the protocol described by Paeshuyse J. et al. (Biochem. Biophys. Res. Commun. 348: 139-144, 2006). A logarithmic culture of HCV replicon-carrying cells was diluted in complete DMEM medium supplemented with 250 µg/ml G418 to the concentration of 5-7 x 10³ cells per ml and aliquoted into 96-well white microtitration plate with optical bottom (Nunc) (100 µl per well). After 24h at 37°C the medium was removed and dilution of inhibitors in the range of 5 to 200 nM was added in 100 µl of fresh DMEM medium without G418. Huh-7 cells with 1% DMSO were used as a control. After 4 days at 37°C the medium was removed and 40 µl of mixture of Glo Lysis buffer and Bright-Glo luciferase assay system (Promega) was added to each well. After 2 mins of incubation luminescence was measured with a Synergy HTi (Biotek) device. The experiment was repeated at least three times with three repetitions for each inhibitor concentration. EC₅₀ value (50% effective concentration) was calculated as the concentration of the inhibitor that reduced by 50% the luminescence corresponding to the HCV RNA replication level.

When determining cytotoxicity, as in the antiviral test, logarithmic culture of HCV replicon-carrying cells was diluted in complete DMEM medium supplemented with 250µg/ml G418 to the concentration of 5-7 x 10³ cells per ml and aliquoted into 96-well mictotitration cell culture plate (100 µl per well). After 24h at 37°C medium was removed and inhibitors were added at concentrations of 5 to 5 000 nM in 100 µl of fresh DMEM medium without G418. After 3 days at 37°C the medium was removed and 100 µl of fresh DMEM medium with 0.5 mg/ml of MTT (tetrazolium bromide, Sigma) was added to each well. After 3 to 4 hours of incubation at 37°C the medium with MTT was removed and 100 µl of 0.04 N HCl solution in absolute isopropanol was added to solubilise the precipitated dye. Dye absorbance was measured at 570 nm, while absorbance of the background was measured at 650 nm using Synergy HTi (Biotek). The experiment was repeated at least 3 times with 3 repetitions for each inhibitor concentration. After background substraction CC₅₀ value (50% cytotoxicity) was calculated as the inhibitor concentration that inhibited the cell growth by 50%.

Values of EC₅₀, CC₅₀ and therapeutic index (TI = CC₅₀/EC₅₀) obtained for examined derivatives and parent antibiotics, such as epidoxorubicin and doxorubicin, are shown in Table 3.

**Table 3. EC₅₀±SD, CC₅₀±SD and TI values for selected amidino derivatives of anthracycline antibiotics and the parent antibiotics**

| Number of derivative | EC₅₀ [nM] | ± SD | CC₅₀ [nM] | ± SD | **TI** |
|---|---|---|---|---|---|
| **75** | 8.40 | 2.82 | 47.85 | 13.3 | **5.7** |
| **94** | 56.87 | 3.04 | 1092.45 | 168.24 | **19.2** |
| **125** | 114.04 | 47.73 | 909.49 | 213.5 | **8.0** |
| **54** | 128.58 | 32.45 | 4280.63 | 437.7 | **33.3** |
| **133** | 137.64 | 47.42 | 1567.23 | 508.7 | **11.4** |
| **Doxorubicin** | 42.91 | 8.96 | 332.38 | 72.6 | **7.7** |
| **Epidoxorubicin** | 73.47 | 12.61 | 674.93 | 107.7 | **9.2** |

Example 3. To 210 mL of apyrogene water 100 mg of 3'-deamino-3'-(N,N-3°-methylaza-1",5"-pentamethyleneacetamidino)-epidoxorubicin hydrochloride (**131**), 0.5 g of lactose, 10 mg of nipagin M and 100 mg of sodium chloride were added and mixed until dissolving. Then, the resulting solution was filtered through an aseptic filter and it was dosed 3.5 mL each to 10 mL glass vials and upon freezing to -45°C it was freeze dried. 56 vials for injections comprising:
1.6±0.1 mg of 3'-deamino-3'-(N,N-3"-methylaza-1",5"-pentamethyleneacetamidino)-epidoxorubicin hydrochloride (**131**),
8.3±0.2 mg of lactose,
0.16±0.01 mg of nipagin M,
1.6±0.1 mg of sodium chloride, were obtained.

Example 4. To 210 mL of water, 60 mg of 3'-deamino-3'-(N,N-1°,5°-pentamethyleneacetamidino)-epidaunorubicin hydrochloride (**91**), 0.6 g of lactose, 12 mg of nipagin M and 100 mg of sodium chloride were added and mixed until dissolving. Then, the resulting solution was filtered through an aseptic filter and dosed 3.5 mL each to 10 mL glass vials and Upon freezing to -45°C it was freeze dried. 56 vials for injections comprising:
1.0±0.1 mg of 3'-deamino-3'-(N,N-1",5"-pentamethyleneacetamidino)-epidaunorubicin (**91**),
10.0±0.2 mg of lactose,
0.2±0.01 mg of nipagin **M**,
1.6±0.1 mg of sodium chloride, were obtained.

Example 5. To 210 mL of water, 50 mg of 3'-deamino-3'-(N,N-1°,4°-tetramethyleneformamidino)-epidoxorubicin hydrochloride (**123**), 50 mg of 3'-deamino-3'-(N,N-3"-methylaza-1",5"-pentamethyleneacetamidino)-epidaunorubicin hydrochloride (**94**), 0.75 g of lactose, 15 mg of nipagin **M** and 50 mg of sodium chloride were added and mixed until dissolving. Then, the resulting solution was filtered through an aseptic filter, dosed 3,6 mL each to 10 mL glass vials and upon freezing to -45°C it was freeze dried. 55 vials for injections comprising:
0.85±0.05 mg of 3'-deamino-3'-(N,N-1",4"-tetramethyleneformamidino)-epidoxorubicin hydrochloride (**123**),
0.85t0.05 mg of 3'-deamino-3'-(N,N-3"-methylaza-1",5"-pentamethyleneacetamidino)-epidaunorubicin hydrochloride (**94**),
12.8±0.2 mg of lactose,
0.26±0.01 mg of nipagin bM,
0.85±0.05 mg of sodium chloride, were obtained.

Example 6. To 210 mL of water, 50 mg of doxorubicin hydrochloride, 150 mg of 3'-deamino-3'-(N,N-1",5"-pentamethyleneformamidino)-epidaunorubicin hydrochloride (**73**), 0.75 g of lactose and 15 mg of nipagin M were added and mixed until dissolving. Then, the resulting solution was filtered through an aseptic filter, dosed 3.6 mL each to 10 mL glass vials and upon freezing to -40°C it was freeze dried. 57 vials for injections, comprising:
0.85±0.05 mg of doxorubicin hydrochloride,
2.5±0.1 mg of 3'-deamino-3'-(N,N-1",5"-pentamethyleneformamidino)-epidaunorubicin hydrochloride (**73**),
12.8±0.2 mg of lactose,
0.26±0.01 mg of nipagin M, were obtained.

Example 7. To 230 mL of water, 50 mg of doxorubicin hydrochloride, 100 mg of 3'-deamino-3'-(N,N-1",4"-tetramethyleneformamidino)-doxorubicin hydrochloride (**35**) 50 mg of 3'-deamino-3'-(N,N-3"-methylaza-1",5"-pentamethyleneacetamidino)-epidoxorubicin hydrochloride (**131**), 0.75 g of lactose and 15 mg of nipagin M were added and mixed until dissolving. Then, the resulting solution was filtered through an aseptic filter, dosed 3.7 mL each to 10 mL glass vials. Upon freezing to -45°C it was freeze dried. 58 vials comprising:
0.8±0.05 mg of doxorubicin hydrochloride,
1.6±0.13 mg of 3'-deamino-3'-(N,N-1",4"-tetramethyleneformamidino)-doxorubicin hydrochloride (**35**),
0.8±0.05 mg of 3'-deamino-3'-(N,N-3"-methylaza-1",5"-pentamethyleneacetamidino)-epidoxorubicin hydrochloride (**131**),
12.0±0.2 mg of lactose,
0.24±0.01 mg of nipagin M, were obtained.

## Claims

1. Use of anthracycline antibiotics of general formula 1 or 2, wherein R¹ is hydrogen or methoxy group, R² is hydrogen or hydroxy group, R³ is hydroxy group in an axial or equatorial orientation, R⁴ and R⁶ are hydrogen or methyl group, R⁶ is 1-phenylethyl group or R⁵ and R⁸ together with a nitrogen atom are N,N-diethyl, N,N-dipropyl or N,N-dibutyl group, for R⁵ and R⁶ together with a nitrogen atom form N,N-1",4"-tetramethylene, N,N-3"-oxa-1",5"-pentamethylene, N,N-1",5"-pentamethylene, N,N-1",6"-hexa-methylene, N,N-1",7"-heptamethylene, N,N-3"-methylaza-1",5"-pentamethylene or 1-indolinyl group, whereas if X is HCl molecule, the compound is hydrochloride of a derivative of general formula 1 or if X is 0, the compound is a derivative in a free base form of general formula 1 wherein, these derivatives are used as active ingredients for preparation of a medicament for treatment of viral infections caused by hepatitis C virus (HCV).

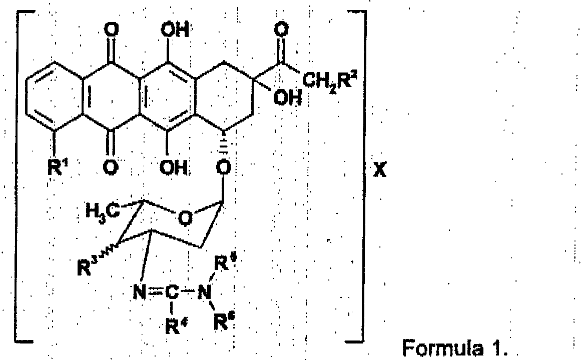

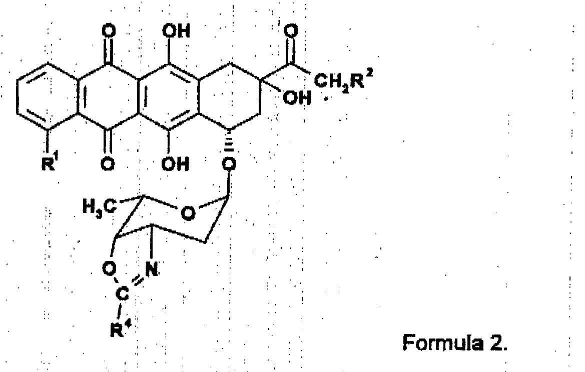

2. Use according to claim 1 wherein the anthracycline antibiotics are selected from the group consisting of:
3'-deamino-3'-(N,N-1",4"-tetramethyleneformamidino)-daunorubicin hydrochloride,
3'-deamino-3'-(N,N-1",4"-tetramethyleneformamidino)-daunorubicin,
3'-deamino-3'-(N,N-3"-oxa-1",5"-pentamethyleneformamidino)-daunorubicin hydrochloride,
3'-deamino-3'-(N,N-3"-oxa-1",5"-pentamethyleneformamidino)-daunorubicin,
3'-deamino-3'-(N,N-1",5"-pentamethyleneformamidino)-daunorubicin hydrochloride,
3'-deamino-3'-(N,N-1",5"-pentamethyleneformamidino)-daunorubicin,
3'-deamino-3'-(N,N-1",6"-hexamethyleneformamidino)-daunorubicin hydrochloride,
3'-deamino-3'-(N,N-3"-methylaza-1",5"-pentamethyleneformamidino)-daunorubicin hydrochloride,
3'-deamino-3'-(N,N-3"-methylaza-1",5"-pentamethyleneformamidino)-daunorubicin,
3'-deamino-3'-(N,N-o-ethylenephenyleneformamidino)-daunorubicin hydrochloride,
3'-deamino-3'-(N,N-o-ethylenephenyleneformamidino)-daunorubicin,
3'-deamino-3'-(N,N-diethylformamidino)-daunorubicin hydrochloride,
3'-deamino-3'-(N,N-diethylformamidino)-daunorubicin,
3'-deamino-3'-(N,N-dipropylformamidino)-daunorubicin hydrochloride,
3'-deamino-3'-(N,N-dipropylformamidino)-daunorubicin,
3'-deamino-3'-(N,N-dibutylformamidino)-daunorubicin hydrochloride,
3'-deamino-3'-(N,N-dibutylformamidino)-daunorubicin,
3'-deamino-3'-(N,N-1",4"-tetramethyleneacetamidino)-daunorubicin hydrochloride,
3'-deamino-3'-(N,N-1",4"-tetramethyleneacetamidino)-daunorubicin,
3'-deamino-3'-(N,N-3"-oxa-1",5"-pentamethyleneacetamidino)-daunorubicin hydrochloride,
3'-deamino-3'-(N,N-3"-oxa-1",5"-pentamethyleneacetamidino)-daunorubicin,
3'-deamino-3'-(N,N-1",5"-pentamethyleneacetamidino)-daunorubicin hydrochloride,
3'-deamino-3'-(N,N-1",5"-pentamethyleneacetamidino)-daunorubicin,
3'-deamino-3'-(N,N-1",6"-hexamethyleneacetamidino)-daunorubicin hydrochloride,
3'-deamino-3'-(N,N-3"-methylaza-1",5"-pentamethyleneacetamidino)-daunorubicin hydrochloride,
3'-deamino-3'-(N,N-3"-methylaza-1",5"-pentamethyleneacetamidino)-daunorubicin,
3'-deamino-3'-(N,N-o-ethylenephenyleneacetamidino)-daunorubicin hydrochloride,
3'-deamino-3'-(N,N-o-ethylenephenylenacetamidino)-daunorubicin,
3'-deamino-3'-(N,N-diethylacetamidino)-daunorubicin hydrochloride,
3'-deamino-3'-(N,N-diethylacetamidino)-daunorubicin,
3'-deamino-3'-(N,N-dipropylacetamidino)-daunorubicin hydrochloride,
3'-deamino-3'-(N,N-dipropylacetamidino)-daunorubicin,
3'-deamino-3'-(N,N-dibutylacetamidino)-daunorubicin hydrochloride,
3'-deamino-3'-(N,N-dibutylacetamidino)-danorubicin,
3'-deamino-3'-(N,N-1",4"-tetramethyleneformamidino)-doxorubicin hydrochloride,
3'-deamino-3'-(N,N-1",4"-tetramethyleneformamidino)-doxorubicin,
3'-deamino-3'-(N,N-3"-oxa-1",5"-pentamethyleneformamidino)-doxorubicin hydrochloride,
3'-deamino-3'-(N,N-3"-oxa-1",5"-pentamethyleneformamidino)-doxorubicin,
3'-deamino-3'-(N,N-1",5"-pentamethyleneformamidino)-doxorubicin hydrochloride,
3'-deamino-3'-(N,N-1",5"-pentamethyleneformamidino)-doxorubicin,
3'-deamino-3'-(N,N-1",6"-hexamethyleneformamidino)-doxorubicin hydrochloride,
3'-deamino-3'-(N,N-3"-methylaza-1",5"-pentamethyleneformamidino)-doxorubicin hydrochloride,
3'-deamino-3'-(N,N-3"-methylaza-1",5"-pentamethyleneformamidino)-doxorubicin,
3'-deamino-3'-(N,N-o-ethylenephenyleneformamidino)-doxorubicin hydrochloride,
3'-deamino-3'-(N,N-o-ethylenephenyleneformamidino)-doxorubicin,
3'-deamino-3'-(N,N-diethylformamidino)-doxorubicin hydrochloride,
3'-deamino-3'-(N,N-diethylformamidino)-doxorubicin,
3'-deamino-3'-(N,N-dipropylformamidino)-doxorubicin hydrochloride,
3'-deamino-3'-(N,N-dipropylformamidino)-doxorubicin,
3'-deamino-3'-(N,N-dibutylformamidino)-doxorubicin hydrochloride,
3'-deamino-3'-(N,N-dibutylformamidino)-doxorubicin,
3'-deamino-3'-(N,N-1",4"-tetramethyleneacetamidino)-doxorubicin hydrochloride,
3'-deamino-3'-(N,N-1",4"-tetramethyleneacetamidino)-doxorubicin,
3'-deamino-3'-(N,N-3"-oxa-1",5"-pentamethyleneacetamidino)-doxorubicin hydrochloride,
3'-deamino-3'-(N,N-3"-oxa-1",5"-pentamethyleneacetamidino)-doxorubicin,
3'-deamino-3'-(N,N-1",5"-pentamethyleneacetamidino)-doxorubicin hydrochloride,
3'-deamino-3'-(N,N-1",5"-pentamethyleneacetamidino)-doxorubicin,
3'-deamino-3'-(N,N-1",6"-hexamethyleneacetamidino)-doxorubicin hydrochloride,
3'-deamino-3'-(N,N-3"-methylaza-1",5"-pentamethyleneacetamidino)-doxorubicin hydrochloride,
3'-deamino-3'-(N,N-3"-methylaza-1",5"-pentamethyleneacetamidino)-doxorubicin,
3'-deamino-3'-(N,N-o-ethylenephenyleneacetamidino)-doxorubicin hydrochloride,
3'-deamino-3'-(N,N-o-ethylenephenyleneacetamidino)-doxorubicin,
3'-deamino-3'-(N,N-diethylacetamidino)-doxorubicin hydrochloride,
3'-deamino-3'-(N,N-diethylacetamidino)-doxorubicin,
3'-deamino-3'-(N,N-dipropylacetamidino)-doxorubicin hydrochloride,
3'-deamino-3'-(N,N-dipropylacetamidino)-doxorubicin
3'-deamino-3'-(N,N-dibuthylacetamidino)-doxorubicin hydrochloride,
3'-deamino-3'-(N,N-dibutylacetamidino)-doxorubicin,
3'-deamino-3'-(N,N-1",4"-tetramethyleneformamidino)-epidaunorubicin hydrochloride,
3'-deamino-3'-(N,N-1",4"-tetramethyleneformamidino)-epidaunorubicin,
3'-deamino-3'-(N,N-3"-oxa-1",5"-pentamethyleneformamidino)-epidaunorubicin hydrochloride,
3'-deamino-3'-(N,N-3"-oxa-1",5"-pentamethyleneformamidino)-epidaunorubicin,
3'-deamino-3'-(N,N-1",5"-pentamethyleneformamidino)-epidaunorbicin hydrochloride,
3'-deamino-3'-(N,N-1",5"-pentamethyleneformamidino)-epidaunorubicin,
3'-deamino-3'-(N,N-1",6"-hexamethyleneformamidino)-epidaunorubicin hydrochloride,
3'-deamino-3'-(N,N-3"-methylaza-1",5"-pentamethyleneformamidino)-epidaunorubicin hydrochloride,
3'-deamino-3'-(N,N-3"-methylaza-1",5"-pentamethyleneformamidino)-epidaunorubicin,
3'-deamino-3'-(N,N-o-ethylenephenyleneformamidino)-epidaunorubicin hydrochloride,
3'-deamino-3'-(N,N-o-ethylenephenyleneformamidino)-epidaunorubicin,
3'-deamino-3'-(N,N-diethylformamidino)-epidaunorubicin hydrochloride,
3'-deamino-3'-(N,N-diethylformamidino)-epidaunorubicin,
3'-deamino-3'-(N,N-dipropylformamidino)-epidaunorubicin hydrochloride,
3'-deamino-3'-(N,N-dipropylformamidino)-epidaunorubicin,
3'-deamino-3'-(N,N-dibutylformamidino)-epidaunorubicin hydrochloride,
3'-deamino-3'-(N,N-dibutylformamidino)-epidaunorubicin, 3'-deamino-3'-(N,N-dibutylformamidino)-epidaunorubicin,
3'-deamino-3'-[N-methyl-N-(1"-phenylethyl)-formamidino]-epidaunorubicin hydrochloride,
3'-deamino-3'-(N,N-1",4"-tetramethyleneacetamidino)-epidaunorubicin hydrochloride,
3'-deamino-3'-(N,N-1",4"-tetramethyleneacetamidino)-epidaunorubicin,
3-deamino-3'-(N,N-3"-oxa-1",5"-pentamethyleneacetamidino)-epidaunorubicin hydrochloride,
3'-deamino-3'-(N,N-3"-oxa-1",5"-pentamethyleneacetamidino)-epidaunorubicin,
3'-deamino-3'-(N,N-1",5"-pentamethyleneacetamidino)-epidaunorubicin hydrochloride,
3'-deamino-3'-(N,N-1",5"-pentamethyleneacetamidino)-epidaunorubicin,
3'-deamino-3'-(N,N-1",6"-pentamethyleneacetamidino)-epidaunorubicin hydrochloride,
3'-deamino-3'-(N,N-3"-methylaza-1',5"-pentamethyleneacetamidino)-epidaunorubicin hydrochloride,
3'-deamino-3'-(N,N-3"-methylaza-1',5"-pentamethyleneacetamidino)-epidaunorubicin,
3'-deamino-3'-(N,N-o-ethylenephenyleneacetamidino)-epidaunorubicin hydrochloride;
3'-deamino-3'-(N,N-o-ethylenephenyleneacetamidino)-epidaunorubicin,
3'-deamino-3'-(N,N-diethyleneacetamidino)-epidaunorubicin hydrochloride,
3'-deamino-3'-(N,N-diethylacetamidino)-epidaunorubicin,
3'-deamino-3'-(N,N-dipropylacetamidino)-epidaunorubicin hydrochloride,
3'-deamino-3'-(N,N-dipropylacetamidino)-epidaunorubicin,
3'-deamino-3'-(N,N-dibutylacetamidino)-epidaunorubicin hydrochloride,
3'-deamino-3'-(N,N-dibutylacetamidino)-epidaunorubicin,
3'-deamino-3'-[N-methyl-N-(1"-phenylethyl)-acetamidino]-epidaunorubicin hydrochloride,
3'-deamino-3'-(N,N-1",4"-tetramethyleneformamidino)-epidoxorubicin hydrochloride,
3'-deamino-3'-(N,N-1",4"-tetramethyleneformamidino)-epidoxorubicin,
3'-deamino-3'-(N,N-3"-oxa-1",5"-pentamethyleneformamidino)-epidoxorubicin hydrochloride,
3'-deamino-3'-(N,N-3"-oxa-1",5"-pentamethyleneformamidino)-epidoxorubicin
3'-deamino-3'-(N,N-1",5"-pentamethyleneformamidino)-epidoxorubicin hydrochloride,
3'-deamino-3'-(N,N-1",6"-pentamethyleneformamidino)-epidoxorubicin,
3'-deamino-3'-(N,N-1",6"-hexamethyleneformamidino)-epidoxonibicin hydrochloride,
3'-deamino-3'-(N,N-3"-methylaza-1",5"-pentamethyleneformamidino)-epidoxorubicin hydrochloride
3'-deamino-3'-(N,N-3"-methylaza-1",5"-pentamethyleneformamidino)-epidoxorubicin,
3'-deamino-3'-(N,N-o-ethylenephenyleneformamidino)-epidoxorubicin hydrochloride,
3'-deamino-3'-(N,N-o-ethylenephenyleneformamidino)-epidoxorubicin,
3'-deamino-3'-(N,N-diethylformamidino)-epidoxorubicin hydrochloride,
3'-deamino-3'-(N,N-diethylformamidino)-epidoxorubicin.
3'-deamino-3'-(N,N-dipropylacetamidino)-epidoxorubicin hydrochloride,
3'-deamino-3'-(N,N-dipropylacetamidino)-epidoxorubicin,
3'-deamino-3'(N,N-dibutylacetamidino)-epidoxorubicin hydrochloride,
3'-deamino-3'(N,N-dibutylacetamidino)-epidoxorubicin,
3'-deamino-3-[N-methyl-N-(1"-phenylethyl)-formamidino]-epidoxorubicin. hydrochloride,
3'-deamino-3'-(N,N-1",4"-tetramethyleneacetamidino)-epidoxorubicin hydrochloride,
3'-deamino-3'-(N,N-1",4"-tetramethyleneacetamidino)-epidoxorubicin,
3'-deamino-3'-(N,N-3"-oxa-1",5"-pentamethyleneacetamidino)-epidoxorubicin hydrochloride,
3'-deamino-3'-(N,N-3"-oxa-1" 5"-pentamethyleneacetamidino)-epidoxorubicin
3'-deamino-3'-(N,N-1",5"-pentamethyleneacetamidino)-epidoxorubicin hydrochloride,
3'-deamino-3'-(N,N-1",5"-pentamethyleneacetamidino)-epidoxorubicin,
3'-deamino-3'-(N,N-1"-6"-hexamethylaneacetamidino)-epidoxorubicin hydrochloride.
3'-deamino-3'-(N,N-1",7"-heptamethyleneacetamidino)-epidoxorubicin hydrochloride,
3'-deamino-3'-(N,N-3"-methylaza-1",5"-pentamethyleneacetamidino)-epidoxorubicin hydrochloride,
3'-deamino-3'-(N,N-3"-methylaza-1",5"-pentamethyleneacatamidino)-epidoxorubicin,
3'-deamino-3'-(N,N-o-ethylenephenyleneacetamidino)-epidoxorubicin hydrochloride,
3'-deamino-3'-(N,N-o-ethylenephenyleneacetamidino)-epidoxorubicin;
3'-deamino-3'-(N,N-diethylacetamido)-epidoxorubicin hydrochloride,
3'-deamino-3'-(N,N-diethylacetamidino)-epidoxorubicin,
3'-deamino-3'-(N,N-dipropylacetamidino)-epidoxorubicin hydrochloride,
3'-deamino-3'-(N,N-dipropylacetamidino)-epidoxorubicin,
3'-deamino-3'-(N,N-dibutylacetamidino)-epidoxorubicin hydrochloride,
3'-deamino-3'-(N,N-dibutylacetamidino)-epidoxorubicin,
3'-deamino-3'-[N-methyl-N-(1"-phenylethyl)-acetamidino]epidoxorubicin hydrochloride,
3'-deamino-3'-N,4'-O-methylidenedaunorubicin,
3'-deamino-3'-N,4'-O-methylidenedoxorubicin,
3'-deamino-3'-N,4'-O-ethylidenedaunorubicin,
3'-deamino-3'-N, 4'-O-ethylidenedoxorubicin:

3. Use according to Claim 1 wherein anthracycline antibiotics are inhibitors of NS3 helicase of hepatitis C virus (HCV) and are used for the preparation of a medicament for inhibiting viral replication.

4. Use according to Claim 3 wherein anthracycline antibiotics are administered in an effective dosage to inhibit viremia, caused by hepatitis C virus replication.

5. Pharmaceutical compositions comprising pharmaceutically acceptable carriers, diluents, excipients and anthracycline antibiotic/s of .general
formula 1 or 2, as active ingredients, wherein R¹ is hydrogen or methoxy group, R² is hydrogen or hydroxy group, R³ is hydroxy group in an axial or equatorial orientation, R⁴ and R⁵ are hydrogen or methyl group, R⁶ is 1-phenylethyl group or R⁵ and R⁶ together with a nitrogen atom are N,N-diethyl, N,N-dipropyl or N,N-dibutyl group, or R⁵ and R⁶ together with a nitrogen atom form N,N-1",4"-tetramethylene, N,N-3"-oxa-1",5"-pentamethylene, N,N-1",5"-pentamethylene, N,N-1",6"-hexamethylene, N,N-1",7"-heptamethylene, N,N-3"-methylaza-1",5"-pentamethylene or 1-indolinyl group, whereas if X is HCl molecule, the compound is hydrochloride of a derivative of general formula 1 or if X is 0, the compound is a derivative in a free base form of general formula 1 for use in the treatment of infection caused by hepatitis C virus.

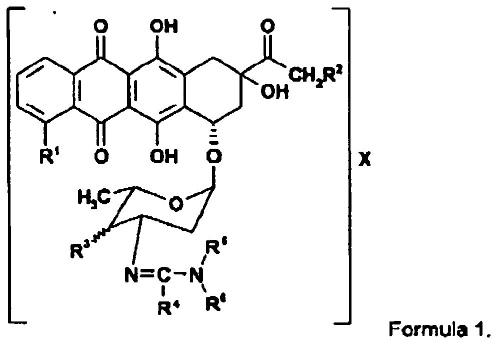

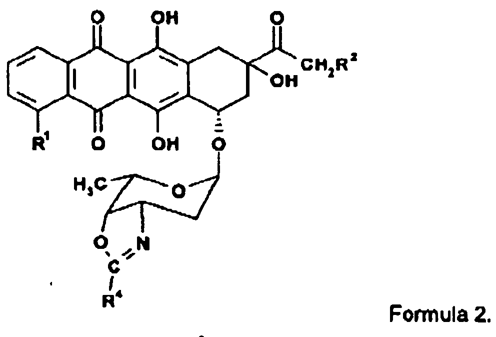

6. Pharmaceutical compositions for use according to claim 5 in combination with a parent anthracycline antibiotic or another antiviral drug.

## Patentansprüche

1. Verwendung von Anthracyclin-Antibiotika der allgemeinen Formel 1 oder 2, worin R¹ Wasserstoff oder Methoxy-Gruppe ist, R² Wasserstoff oder eine Hydroxy-Gruppe ist, R³ eine Hydroxy-Gruppe in einer axialen oder äquatorialen Orientierung ist, R⁴ und R⁵ Wasserstoff oder eine Methylgruppe sind, R⁶ 1 -Phenethylgruppe oder R⁵ und R⁶ zusammen mit einem Stickstoffatom N,N-Diethyl-, N,N-Dipropyl- oder N, N-Dibutyl-Gruppe sind, oder R⁵ und R⁶ zusammen mit einem Stickstoffatom N,N-1",4"-Tetramethylen, N,N-3"-Oxa-1",5"-Pentamethylen, N,N-1",5"-Pentamethylen, N,N-1",6" -Hexamethylen, N,N-1",7"-Heptamethylen, N,N-3"-Methylaza-1",5"-Pentamethylen oder 1-Indolinyl-Gruppe bilden, wohingegen, wenn X für HCl-Molekül steht, ist die Verbindung Hydrochlorid von Derivat der allgemeinen Formel 1 oder, wenn X 0 ist, ist die Verbindung ein Derivat in Form der freien Base der allgemeinen Formel 1, worin diese Derivate als Wirkstoffe zur Herstellung eines Medikaments zur Behandlung von viralen Infektionen, die durch Hepatitis-C-Virus (HCV) verursacht werden.

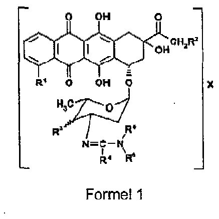

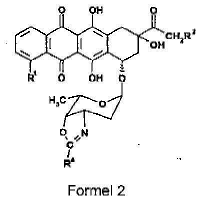

2. Verwendung nach Anspruch 1, worin die Anthracyclin-Antibiotika aus der Gruppe ausgewählt sind, die aus den folgenden Verbindungen besteht:
3'-Deamino-3'-(N,N-1",4"-tetramethylenformamidino)-daunorubicin Hydrochlorid,
3'-Deamino-3'-(N,N-1",4"-tetramethylenformamidino)-daunorubicin,
3'-Deamino-3'-(N,N-3"-oxa-1",5"-pentamethylenformamidino)-daunorubicin Hydrochlorid,
3'-Deamino-3'-(N,N-3"-oxa-1",5"-pentamethylenformamidino)-daunorubicin,
3'-Deamino-3'-(N,N-1",5"-pentamethylenformamidino)-daunorubicin Hydrochlorid,
3'-Deamino-3'-(N,N-1",5"- pentamethylenformamidino)-daunorubicin,
3'-Deamino-3'-(N,N-1",6"-hexamethylenformamidino)-daunorubicin Hydrochlorid,
3'-Deamino-3'-(N,N-3"-methylaza-1 ",5"-pentamethylenformamidino)-daunorubicin Hydrochlorid,
3'-Deamino-3'-(N,N-3"-methylaza-1",5"-pentamethylenformamidino)- daunorubicin,
3'-Deamino-3'-(N,N-o-ethylenphenylenformamidino)-daunorubicin Hydrochlorid,
3'-Deamino-3'-(N,N-o-ethylenphenylenformamidino)-daunorubicin,
3'-Deamino-3'-(N,N-diethylformamidino)-daunorubicin Hydrochlorid,
3'-Deamino-3'-(N,N-diethylformamidino)-daunorubicin,
3'-Deamino-3'-(N,N-dipropylformamidino)-daunorubicin Hydrochlorid,
3'-Deamino-3'-(N,N-dipropylformamidino)-daunorubicin,
3'-Deamino-3'-(N,N-dibutylformamidino)-daunorubicin Hydrochlorid,
3'-Deamino-3'-(N,N-dibutylformamidino)-daunorubicin,
3'-Deamino-3'-(N,N-1",4"-tetramethylenacetamidino)-daunorubicin Hydrochlorid,
3'-Deamino-3'-(N,N-1",4"-tetramethylenacetamidino)-daunorubicin,
3'-Deamino-3'-(N,N-3"-oxa-1",5"-pentamethylenacetamidino)-daunorubicin Hydrochlorid,
3'-Deamino-3'-(N,N-3"-oxa-1",5"-pentamethylenacetamidino)-daunorubicin,
3'-Deamino-3'-(N,N-1",5"-pentamethylenacetamidino)-daunorubicin Hydrochlorid,
3'-Deamino-3'-(N,N-1",5"-pentamethylenacetamidino)-daunorubicin,
3'-Deamino-3'-(N,N-1",6"-hexamathylenacetamidino)-daunorubicin Hydrochlorid.
3'-Deamino-3'-(N,N-3"-methylaza-1",5"-pentamethylenacetamidino)-daunorubicin Hydrochlorid,
3'-Deamino-3'-(N,N-3"-methylaza-1",5"-pentamethylenacetamidino)- daunorubicin,
3'-Deamino-3'-(N,N-o-ethylenphenylenacetamidino)-daunorubicin Hydrochlorid,
3'-Deamino-3'-(N,N-o-ethylenphenylenacetamidino)-daunorubicin.
3'-Deamino-3'-(N,N-diethylacetamidino)-daunorubicin Hydrochlorid,
3'-Deamino-3'-(N,N-diethylacetamidino)-daunorubicin,
3'-Deamino-3'-(N,N-dipropylacetamidino)-daunorubicin Hydrochlorid,
3'-Deamino-3'-(N,N-dipropylacetamidino)-daunorubicin,
3'-Deamino-3'-(N,N-dibutylacetamidino)-daunorubicin Hydrochlorid,
3'-Deamino-3'-(N,N-dibutylacetamidino)-daunorubicin,
3'-Deamino-3'-(N,N-1",4"-tetramethylenformamidino)-doxorubicin Hydrochlorid,
3'-Deamino-3'-(N,N-1",4"-tetramethylenformamidino)-doxorubicin,
3'-Deamino-3'-(N,N-3"-oxa-1",5"-pentamethylenformamidino)-doxorubicin Hydrochlorid,
3'-Deamino-3'-(N,N-3"-oxa-1",5"-pentamethylenformamidino)-doxorubicin,
3'-Deamino-3'-(N,N-1",5"-pentamethylenformamidino)-doxorubicin Hydrochlorid,
3'-Deamino-3'-(N,N-1",5"-pentamethyleneformamidino)-doxorubicin,
3'-Deamino-3'-(N,N-1",6"-hexamethylenformamidino)-doxorubicin Hydrochlorid,
3'-Deamino-3'-(N,N-3"-methylaza-1",5"-pentamethylenformamidino)-doxorubicin Hydrochlorid,
3'-Deamino-3'-(N,N-3"-methylaza-1",5"-pentamethylenformamidino)- doxorubicin,
3'-Deamino-3'-(N,N-o-ethylenphenylenformamidino)-doxorubicin Hydrochlorid,
3'-Deamino-3'-(N,N-o-ethylenphenylenformamidino)-doxorubicin,
3'-Deamino-3'-(N,N-diethylformamidino)-doxorubicin Hydrochlorid,
3'-Deamino-3'-(N,N-diethylformamidino)-doxorubicin,
3'-Deamino-3'-(N,N-dipropylformamidino)-doxorubicin Hydrochlorid,
3'-Deamino-3'-(N,N-dipropylformamidino)-doxorubicin,
3'-Deamino-3'-(N,N-dibutylformamidino)-doxorubicin Hydrochlorid,
3'-Deamino-3'-(N,N-dibutylformamidino)-doxorubicin,
3'-Deamino-3'-(N,N-1",4"-tetramethylenacetamidino)-doxorubicin Hydrochlorid,
3'-Deamino-3'-(N,N-1 ",4"-tetramethylenacetamidino)-doxorubicin,
3'-Deamino-3'-(N,N-3"-oxa-1",5"-pentamethylenacetamidino)-doxorubicin Hydrochlorid,
3'-Deamino-3'-(N,N-3"-oxa-1",5"-pentamethylenacetamidino)-doxorubicin,
3'-Deamino-3'-(N,N-1",5"-pentamethylenacetamidino)-doxorubicin Hydrochlorid,
3'-Deamino-3'-(N,N-1",5"-pentamethylenacetamidino)-doxorubicin,
3'-Deamino-3'-(N,N-1",6"-hexamethylenacetamidino)-doxorubicin Hydrochlorid,
3-Deamino-3'-(N,N-3"-methylaza-1",5"-pentamethylenacetamidino)-doxorubicin Hydrochlorid,
3'-Deamino-3'-(N,N-o-ethylenphenylenacetamidino)-doxorubicin Hydrochlorid,
3'-Deamino-3'-(N,N-o-ethylenphenylenacetamidino)-doxorubicin,
3'-Deamino-3'-(N,N-diethylacetamidino)-doxorubicin Hydrochlorid,
3'-Deamino-3'-(N,N-diethylacetamidino)- doxorubicin,
3'-Deamino-3'-(N,N-dipropylacetamidino)-doxorubicin Hydrochlorid,
3'-Deamino-3'-(N,N-dipropylacetamidino)-doxorubicin,
3'-Deamino-3'-(N,N-dibutylacetamidino)-doxorubicin Hydrochlorid,
3'-Deamino-3'-(N,N-dibutylacetamidino)-doxorubicin,
3'-Deamino-3'-(N,N-1",4"-tetramethylenformamidino)-epidaunorubicin Hydrochlorid,
3'-Deamino-3'-(N,N-1",4"-tetramethylenformamidino)-epidaunorubicin,
3'-Deamino-3'-(N,N-3"-oxa-1",5"-pentamethylenformamidino)-epidaunorubicin Hydrochlorid,
3'-Deamino-3'-(N,N-3"-oxa-1",5"-pentamethylenformamidino)-epidaunorubicin,
3'-Deamino-3'-(N,N-1",5"-pentamethylenformamidino)-epidaunorubicin Hydrochlorid,
3'-Deamino-3'-(N,N-1",5"-pentamethylenformamidino)-epidaunorubicin,
3'-Deamino-3'-(N,N-1 ",6"-hexamethylenformamidino)-epidaunorubicin Hydrochlorid,
3'-Deamino-3'-(N,N-3"-methylaza-1",5"-pentamethylenformamidind)-epidaunorubicin Hydrochlorid,
3'-Deamino-3'-(N,N-3"-methylaza-1",5"-pentamethylenformamidino)-epidaunorubicin,
3'-Deamino-3'-(N,N-o-ethylenphenylenformamidino)-epidaunorubicin Hydrochlorid,
3'-Deamino-3'-(N,N-o-ethylenphenylenformamidino)-epidaunorubicin,
3'-Deamino-3'-(N,N-diethylformamidino)-epidaunorubicin Hydrochlorid,
3'-Deamino-3'-(N,N-diethylformamidino)-epidaunorubicin,
3'-Deamino-3'-(N,N-dipropylformamidino)-epidaunorubicin Hydrochlorid,
3'-Deamino-3'-(N,N-dipropylformamidino)-epidaunorubicin,
3'-Deamino-3'-(N,N-dibutylformamidino)-epidaunorubicin Hydrochlorid,
3'-Deamino-3'-(N,N-dibutylformamidino)-epidaunorubicin,
3'-Deamino-3'-[N-methyl-N-(1"-phenylethyl)-formamidino]-epidaunorubicin Hydrochlorid,
3'-Deamino-3'-(N,N-1",4"-tetramethylenacetamidino)-epidaunorubicin Hydrochlorid,
3'-Deamino-3'-(N,N-1",4"-tetramethylenacetamidino)-epidaunorubicin,
3'-Deamino-3'-(N,N-3"-oxa-1",5"-pentamethylenacetamidino)-epidaunorubicin Hydrochlorid,
3'-Deamino-3'-(N,N-3"-oxa-1",5"-pantamethylenacetamidino)- epidaunorubicin,
3'-Deamino-3'-(N,N-1",5"-pentamethylenacetamidino)-epidaunorubicin Hydrochlorid,
3'-Deamino-3'-(N, N-1",5"-pentamethylenacetamidino)-epidaunorubicin,
3'-Deamino-3'-(N,N-1",6"-hexamethylenacetamidino)-epidaunorubicin Hydrochlorid,
3'-Deamino-3'-(N,N-3"-methylaza-1",5"-pentamethylenacetamidino)-epidaunorubicin Hydrochlorid,
3'-Deamino-3'-(N,N-3"-methylaza-1",5"-pentamethylenacetamidino)-epidaunorubicin,
3'-Deamino-3'-(N,N-o-ethylenephenylenacetamidino)-epidaunorubicin Hydrochlorid,
3'-Deamino-3'-(N,N-o-ethylenephenylenacetamidino)-epidaunorubicin,
3'-Deamino-3'-(N,N-diethylacetamidino)-epidaunorubicin Hydrochlorid,
3'-Deamino-3'-(N,N-diethylacetamidino)-epidaunorubicin,
3'-Deamino-3'-(N,N-dipropylacetamidino)-epidaunorubicin Hydrochlorid,
3'-Deamino-3'-(N,N-dipropylacetamidino)-epidaunorubicin,
3'-Deamino-3'-(N,N-dibutylacetamidino)-epidaunorubicin Hydrochlorid.
3'-Deamino-3'-(N,N-dibutylacetamidino)-epidaunorubicin,
3'-Deamino-3'-[N-methyl-N-(1"-phenylethyl)-acetamidino]-epidaunorubicin Hydrochlorid,
3'-Deamino-3'-(N,N-1",4"-tetramethylenformamidino)-epidoxorubicin Hydrochlorid,
3'-Deamino-3'-(N,N-1",4"-tetramethylenformamidino)-epidoxorubicin,
3'-Deamino-3'-(N,N-3"-oxa-1",5"-pentamethylenformamidino)-epidoxorubicin) Hydrochlorid,
3'-Deamino-3'-(N,N-3"-oxa-1",5"-pentamethylenformamidino)-epidoxorubicin,
3'-Deamino-3'-(N,N-1 ",5"-pentamethylenformamidino)-epidoxorubicin Hydrochlorid,
3'-Deamino-3'-(N,N-1",5"-pentamethylenformamidino)-epidoxorubicin,
3'-Deamino-3'-(N,N-1",6"-hexamethylenformamidino)-epidoxorubicin Hydrochlorid,
3'-Deamino-3'-(N,N-3"-methylaza-1",5"-pentamethylenformamidino)-epidoxorubicin Hydrochlorid,
3'-Deamino-3'-(N,N-3"-methylaza-1 ",5"-pentamethylenformamidino)-epidoxorubicin,
3'-Deamino-3'-(N,N-o-ethylenphenylenformamidino)-epidoxorubicin Hydrochlorid,
3'-Deamino-3'-(N,N-o-ethylenphenylenformamidino)-epidoxorubicin,
3'-Deamino-3'-(N,N-diethylformamidino)-epidoxorubicin Hydrochlorid,
3'-Deamino-3'-(N,N-diethylformamidino)-epidoxorubicin,
3'-Deamino-3'-(N,N-dipropylacetamidino)-epidoxorubicin Hydrochlorid,
3'-Deamino-3'-(N.N-dipropylacetamidino)-epidoxorubicin,
3'-Deamino-3'-(N,N-dibutylacetamidino)-epidoxorubicin Hydrochlorid,
3'-Deamino-3'-(N,N-dibutylacetamidino)-epidoxorubicin,
3'-Deamino-3'-[N-methyl-N-(1"-phenylethyl)-formamidino]-epidoxorubicin Hydrochlorid,
3'-Deamino-3'-(N,N-1",4"-tetramethylenacetamidino)-epidoxorubicin Hydrochlorid,
3'-Deamino-3'-(N,N-1",4"-tetramethylenacetamidino)-epidoxorubicin,
3'-Deamino-3'-(N,N-3"-oxa-1",5"-pentamethylenacetamidino)-epidoxorubicin Hydrochlorid,
3'-Deamino-3'-(N,N-3"-oxa-1",5"-pentamethylenacetamidino)-epidoxorubicin
3'-Deamino-3'-(N,N-1",5"-pentamethylenacetamidino)-epidoxorubicin Hydrochlorid,
3'-Deamino-3'-(N,N-1",5"-pentamethylenacetamidino)-epidoxorubicin,
3'-Deamino-3'-(N,N-1",6"-hexamethylenacetamidino)-epidoxorubicin Hydrochlorid,
3'-Deamino-3'-(N,N-1",7"-heptamethylenacetamidino)-epidoxorubicin Hydrochlorid,
3'-Deamino-3'-(N,N-3"-methylaza-1",5"-pentamethylenacetamidino)-epidoxorubicin Hydrochlorid,
3'-Deamino-3'-(N,N-3"-methylaza-1,5"-pentamethylenacetamidino)-epidoxorubicin,
3'-Deamino-3'-(N,N-o-ethylenphenylenacetamidino)-epidoxorubicin Hydrochlorid,
3'-Deamino-3'-(N,N-o-ethylenphenylenacetamidino)-epidoxorubicin,
3'-Deamino-3'-(N,N-diethylacetamidino)-epidoxorubicin Hydrochlorid,
3'-Deamino-3'-(N,N-diethylacetamidino)-epidoxorubicin,
3'-Deamino-3'-(N,N-dipropylacetamidino)-epidoxorubicin Hydrochlorid,
3'-Deamino-3'-(N,N-dipropylacetamidino)-epidoxorubicin,
N-methyl-N-(1"-phenylethyl)-acetamidino]-epidoxorubicin Hydrochlorid,
3'-Deamino-3'-N, 4'-O-methylidendaunorubicin,
3'-Deamino-3'-N, 4'-O-methylidendoxorubicin,
3'-Deamino-3'-N, 4'-O-ethylidendaunorubicin,
3'-Deamino-3'-N, 4'-O-ethylidendoxorubicin.

3. Verwendung nach Anspruch 1, wobei Anthracyclin-Antibiotika Inhibitoren der NS3 Helikase von Hepatitis C Virus (HCV) sind und werden zur Herstellung eines Medikaments zur Hemmung der viralen Replikation verwendet.

4. Verwendung nach Anspruch 3, wobei die Anthracyclin-Antibiotika in einer wirksamen Dosis zur Hemmung der durch Hepatitis C-Virus verursachten Virämie verabreicht sind.

5. Pharmazeutische Zusammensetzungen, umfassend pharmazeutisch akzeptable Träger, Verdünnungsmittel, Hilfsstoffe und Anthracyclin-Antibiotika der allgemeinen Formel 1 oder 2 als aktive Inhaltsstoffe, worin R¹ Wasserstoff oder Methoxy-Gruppe ist, R² Wasserstoff oder eine Hydroxy-Gruppe ist, R³ eine Hydroxy-Gruppe in einer axialen oder äquatorialen Orientierung ist, R⁴ und R⁵ Wasserstoff oder eine Methylgruppe sind, R⁶ 1 -Phenethylgruppe oder R⁵ und R⁶ zusammen mit einem Stickstoffatom N,N-Diethyl-, N,N-Dipropyl- oder N, N-Dibutyl-Gruppe sind, oder R⁵ und R⁶ zusammen mit einem Stickstoffatom N,N-1",4"-Tetramethylen, N,N-3"-Oxa-1",5"-Pentamethylen, N,N-1",5"-Pentamethylen, N,N-1",6"-Hexamethylen, N,N-1",7"-Heptamethylen, N,N-3"-Methylaza-1",5"-Pentamethylen oder 1-Indolinyl-gruppe bilden, wohingegen, wenn X für HCl-Molekül steht, ist die Verbindung Hydrochlorid von Derivat der allgemeinen Formel 1 oder, wenn X 0 ist, ist die Verbindung ein Derivat in Form der freien Base der allgemeinen Formel 1, zur Verwendung bei der Behandlung von einer Infektion, die durch Hepatitis-C-Virus verursacht wird.

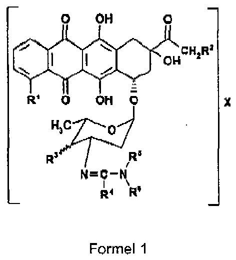

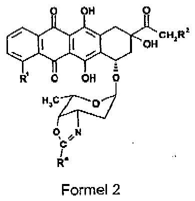

6. Pharmazeutische Zusammensetzungen zur Verwendung nach Anspruch 5 in Kombination mit Ausgangs-Anthracyclin-Antibiotikum oder einem anderen antiviralen Arzneimittel.

## Revendications

1. Utilisation des antibiotiques anthracyclines de la formule générale 1 ou 2, où R¹ est hydrogène ou un groupe méthoxy, R² est hydrogène ou un groupe hydroxy, R³ est un groupe hydroxy dans une orientation axiale ou équatoriale, R⁴ et R⁵ sont des atomes d'hydrogène ou des groupes méthyles, R⁶ est un groupe 1-phényléthyle ou R⁵ et R⁶ avec un atome d'azote sont des groupes N,N-diéthyle, N,N-dipropyle ou N,N-dibutyle, ou R⁵ et R⁶ avec un atome d'azote forment N,N-1"4"-tetraméthylène, N,N-3"-oxa-1",5"-pentaméthylène, N,N-1",5"-pentaméthylène, N,N-1",6"-hexaméthylène. N,N-1",7"-heptaméthylène, N,N-3"-méthylaza-1",5"-pentaméthylène ou un group 1-indolinyle, considérant que si X est une molécule de HCl, le composé est le chlorhydrate d'un dérivé de la formule générale 1 ou si X est O, le composé est un dérivé dans une forme de base libre de la formule générale 1, où ces dérivés sont utilisés comme ingrédients actifs pour la préparation d'un médicament pour le traitement des infections virales causées par le virus de l'hépatite C (VHC).

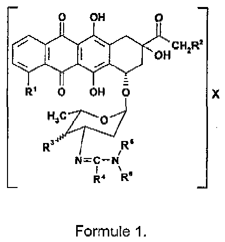

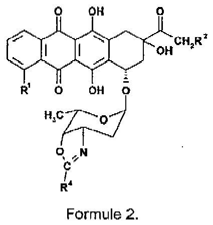

2. Utilisation selon la revendication 1, dans lequel des antibiotiques anthracyclines sont sélectionnés parmi le groupe constitué de:
chlorhydrate de 3'-désamino-3'-(N,N-1",4"-tetraméthylèneformamidine)-daunorubicine,
3'-désamino-3'-(N,N-1",4"-tetraméthylèneformamidine)-daunorubicine,
chlorhydrate de 3'-désamino-3'-(N,N-3"-oxa-1",5"-pentaméthylèneformamidine)-daunorubicine,
3'-désamino-3'-(N,N-3"-oxa-1",5"-pentaméthyléneformamidine)-daunorubicine,
chlorhydrate de 3'-désamino-3'-(N,N-1",5"-pentaméthylèneformamidine)-daunorubicine,
3'-désamino-3'-(N,N-1",5"-pentaméthylèneformamidine)-daunorubicine,
chlorhydrate de 3'-désamino-3'-(N,N-1 ",6"-hexaméthylèneformamidine)-daunorubicine,
chlorhydrate de 3'-désamino-3'-(N,N-3"-méthylaza-1",5"-pentaméthylèneformamidine)-daunorubicine,
3'-désamino-3'-(N,N-3"-méthylaza-1",5"-pentaméthylèneformamidine)-daunorubicine,
chlorhydrate de 3'-désamino-3'-(N,N-o-éthylènephényleneformamidine)-daunorubicine,
3'-désamino-3'-(N,N-o-éthylène-phényleneformamidine)-daunorubicine,
chlorhydrate de 3'-désamino-3'-(N,N-diéthylformamidine)-daunorubicine, 3'-désamino-3'-(N,N-diéthylformamidine)-daunorubicine,
chlorhydrate de 3'-désamino-3'-(N,N-dipropylformamidine)-daunorubicine, 3'-désamino-3'-(N,N-dipropylformamidine)-daunorubicine,
chlorhydrate de 3'-désamino-3'-(N,N-dibutylformamidine)-daunorubicine, 3'-désamino-3'-(N,N-dibutylformamidine)-daunorubicine,
chlorhydrate de 3'-désamino-3'-(N,N-1",4"-tetraméthylèneacétamidine)-daunorubicine,
3'-désamino-3'-(N,N-1",4"-tetramethylèneacétamidine)-daunorubicine,
chlorhydrate de 3'-désamino-3'-(N,N-3"-oxa-1",5"-pentaméthylèneacétamidine)-daunorubicine,
3'-désamino-3'-(N,N-3"-oxa-1",5"-pentaméthyléneacétamidine)-daunorubicine,
chlorhydrate de 3'-désamino-3'-(N,N-1",5"-pentaméthylèneacétamidine)-daunorubicine,
3'-désamino-3'-(N,N-1",5"-pentaméthylèneacétamidine)-daunorubicine,
chlorhydrate de 3'-désamino-3'-(N,N-1",6"-hexaméthyléneacétamidine)-daunorubicine,
chlorhydrate de 3'-désamino-3'-(N,N-3"-méthylaza-1",5"-pentaméthylèneacétamidine)-daunorubicine,
3'-désamino-3'-(N,N-3"-méthylaza-1",5"-pentaméthyléneacétamidine)-daunorubicine,
chlorhydrate de 3'-désamino-3'-(N,N-o-éthylènephényleneacétamidine)-daunorubicine,
3'-désamino-3'-(N,N-o-éthylènephényleneacétamidine)-daunorubicine,
chlorhydrate de 3'-désamino-3'-(N,N-diéthylacétamidine)-daunorubicine, 3'-désamino-3'-(N,N-diéthylacétamidine)-daunorubicine,
chlorhydrate de 3'-désamino-3'-(N,N-dipropylacétamidine)-daunorubicine, 3'-désamino-3'-(N,N-dipropylacétamidine)-daunorubicine,
chlorhydrate de 3'-désamino-3'-(N,N-dibutylacétamidine)-daunorubicine, 3'-désamino-3'-(N,N-dibutylacétamidine)-daunorubicine,
chlorhydrate de 3-désamino-3"(N,N-1",4"-tetraméthyléneformamidine)-doxorubicine,
3'-désamino-3'-(N,N-1 ",4"-tetraméthylèneformamidine)-doxorubicine,
chlorhydrate de 3'-désamino-3'-(N,N-3"-oxa-1",5"-pentaméthylèneformamidine)-doxorubicine.
3'-désamino-3'-(N,N-3"-oxa-1",5"-pentaméthylèneformamidine)-doxorubicine,
chlorhydrate de 3'-désamino-3'-(N,N,1",5"-pentaméthylèneformamidine)-doxorubicine,
3'-désamino-3'-(N,N,1",5"-pentaméthylèneformamidine)-doxorubicine,
chlorhydrate de 3'-désamino-3'-(N,N,1",6"-hexaméthylèneformamidine)-doxorubicine,
chlorhydrate de 3'-désamino-3'-(N,N,3"-méthylaza-1 ",5"-pentaméthylèneformamidine)-doxorubicine,
3'-désamino-3'-(N,N,3"-méthylaza-1",5"-pentaméthylèneformamidine)-doxorubicine,
chlorhydrate de 3'-désamino-3'-(N,N-o-éthylènephényleneformamidine)-doxorubicine,
3'-désamino-3'-(N,N-o-éthylène-phényleneformamidine)-doxorubicine,
chlorhydrate de 3'-désamino-3'-(N,N-diéthylformamidine)-doxorubicine, 3'-désamino-3'-(N,N-diéthylformamidine)-doxorubicine,
chlorhydrate de 3'-désamino-3'-(N,N-dipropylformamidine)-doxorubicine, 3'-désamino-3'-(N,N-dipropylformamidine)-doxorubicine,
chlorhydrate de 3'-désamino-3'-(N,N-dibutylformamidine)-doxorubicine, 3'-désamino-3'-(N,N-dibutylformamidine)-doxorubicine,
chlorhydrate de 3'-désamino-3'-(N,N-1",4"-tetraméthylèneacétamidine)-doxorubicine,
3'-désamino-3'-(N,N-1",4"-tetraméthylèneacétamidine)-doxorubicine,
chlorhydrate de 3'-désamino-3'-(N,N-3"-oxa-1",5"-pentaméthylèneacétamidine)-doxorubicine,
3'-désamino-3'-(N,N-3"-oxa-1",5"-pentaméthylèneacétamidine)-doxorubicine,
chlorhydrate de 3'-désamino-3'-(N,N,1",5"-pentaméthylèneacétamidine)-doxorubicine,
3'-désamino-3'-(N,N,1",5"-pentaméthyléneacétamidine)-doxorubicine,
chlorhydrate de 3'-désamino-3'-(N,N,1",6"-hexaméthyléneacétamidine)-doxorubicine,
chlorhydrate de 3'-désamino-3'-(N,N,3"-méthylaza-1",5"-pentaméthylèneacétamidine)-doxorubicine,
3'-désamino-3'-(N,N,3"-méthylaza-1 ",5"-pentaméthylèneacétamidine)-doxorubicine,
chlorhydrate de 3'-désamino-3'-(N,N-o-éthylènephényleneacétamidine)-doxorubicine,
3'-désamino-3'-(N,N-o-éthylènephényleneacétamidine)-doxorubicine,
chlorhydrate de 3'-désamino-3'-(N,N-diéthylacétamidine)-doxorubicine, 3'-désamino-3'-(N,N-diéthylacétamidine)-doxorubicine,
chlorhydrate de 3'-désamino-3'-(N,N-dipropylacétamidine)-doxorubicine, 3'-désamino-3'-(N,N-propyacetamidine)-doxorubicine,
chlorhydrate de 3'-désamino-3'-(N,N-dibutylacétamidine)-doxorubicine, 3'-désamino-3'-(N,N-dibutylacétamidine)-doxorubicine,
chlorhydrate de 3'-désamino-3'-(N,N-1",4"-tetraméthylèneformamidine)-épidaunorubicine,
3'-désamino-3'-(N,N-1",4"-tetraméthylèneformamidine)-épidaunorubicine,
chlorhydrate de 3'-désamino-3'-(N,N-3"-oxa-1",5"-pentaméthylèneformamidine)-épidaunorubicine,
3'-désamino-3'-(N,N-3"-oxa-1",5"-pentaméthylèneformamidine)-épidaunorubicine,
chlorhydrate de 3'-désamino-3'-(N,N-1",5"-pentaméthylèneformamidine)-épidaunorubicine,
3'-désamino-3'-(N,N-1",5"-pentaméthylèneformamidine)-épidaunorubicine,
chlorhydrate de 3'-désamino-3-(N,N-1",6"-hexaméthylèneformamidine)-épidaunorubicine,
chlorhydrate de 3'-désamino-3'-(N,N-3"-méthylaza-1",5"-pentaméthylèneformamidine)-épidaunorubicine,
3'-désamino-3'-(N,N-3"-méthylaza-1",5"-pentaméthylèneformamidine)-épidaunorubicine,
chlorhydrate de 3'-désamino-3'-(N,N-o-éthylènephényleneformamidine)-épidaunorubicine,
3'-désamino-3'-(N,N-o-éthylènephényleneformamidine)-épidaunorubicine,
chlorhydrate de 3'-désamino-3'-(N,N-diéthylformamidine)-épidaunorubicine, 3'-désamino-3'-(N,N-diéthylformamidine)-épidaunorubicine,
chlorhydrate de 3'-désamino-3'-(N,N-dipropylformamidine)-épidaunorubicine, 3'-désamino-3'-(N,N-dipropylformamidine)-épidaunorubicine,
chlorhydrate de 3'-désamino-3'-(N,N-dibutylformamidine)-épidaunorubicine, 3'-désamino-3'-(N,N-dibutylformamidine)-épidaunorubicine,
chlorhydrate de 3'-désamino-3'-[N-méthyl-N-(1"-phényléthyl)formamidine]-épidaunorubicine,
chlorhydrate de 3'-désamino-3'-(N,N-1",4"-tetraméthyléneacétamidine)-épidaunorubicine,
3'-désamino-3'-(N,N-1",4"-tetraméthylèneacétamidine)-épidaunorubicine,
chlorhydrate de 3'-désamino-3'-(N,N-3"-oxa-1",5"-pentaméthylèneacétamidine)-épidaunorubicine,
3'-désamirio-3'-(N,N-3"-oxa-1",5"-pentaméthylèneacétamidine)-épidaunorubicine,
chlorhydrate de 3'-désamino-3'-(N,N-1",5"-pentaméthylèneacétamidine)-épidaunorubicine.
3'-désamino-3'-(N,N-1",5"-pentaméthylèneacétamidine)-épidaunorubicine,
chlorhydrate de 3'-désamino-3'-(N,N-1",6"-hexaméthylèneacétamidine)-épidaunorubicine,
chlorhydrate de 3'-désamino-3'-(N,N-3"-méthylaza-1",5"-pentaméthylèneacétamidine)-épidaunorubicine,
3'-désamino-3'-(N,N-3"-méthylaza-1",5"-pentaméthylèneacétamid ine)-épidaunorubicine,
chlorhydrate de 3'-désamino-3'-(N,N-o-éthylène-phényleneacétamidine)-épidaunorubicine,
3'-désamino-3'-(N,N-o-éthylène-phényleneacétamidine)-épidaunorubicirie,
chlorhydrate de 3'-désamino-3'-(N,N-diéthylacétamidine)-épidaunorubicine, 3'-désamino-3'-(N,N-diéthylacétamidine)-épidaunorubicine,
chlorhydrate de 3'-désamino-3'-(N,N-dipropylacétamidine)-épidaunorubicine, 3'-désamino-3'-(N,N-dipropylacétamidine)-épidaunorubicine,
chlorhydrate de 3'-désamino-3'-(N,N-dibutylacétamidine)-épidaunorubicine.
3'-désamino-3'-(N,N-dibutylacétamidine)-épidaunorubicine,
chlorhydrate de 3'-désamino-3'-[N-méthyl-N-(1"-phényléthylacétamidine]-épidaunorubicine,
chlorhydrate de 3'-désamino-3'-(N,N-1",4"-tetraméthylèneformamidine)-épidoxorubicine,
3'-désamino-3'-(N,N-1",4"-tetraméthylèneformamidine)-épidoxorubicine,
chlorhydrate de 3'-désamino-3'-(N,N-3"-oxa-1",5"-pentaméthylèneformamidine)-épidoxorubicine,
3'-désamino-3'-(N,N-3"-oxa-1",5"-pentaméthylèneformamidine)-épidoxorubicine,
chlorhydrate de 3'-désamino-3'-(N,N,1",5"-pentaméthylèneformamidine)-épidoxorubicine,
3'-désamino-3'-(N,N,1",5"-pentaméthylèneformamidine)-épidoxorubicine,
chlorhydrate de 3'-désamino-3'-(N,N,1",6"-hexaméthylèneformamidine)-épidoxorubicine,
chlorhydrate de 3'-désamino-3'-(N,N,3"-méthylaza-1",5"-pentaméthylèneformamidine)-épidoxorubicine,
3'-désamino-3'-(N,N,3"-méthylaza-1",5"-pentaméthyléneformamidine)-épidoxorubicine,
chlorhydrate de 3'-désamino-3'-(N,N-o-éthylène-phényleneformamidine)-épidoxorubicine.
3'-désamino-3'-(N,N-o-éthylène-phényleneformamidine)-épidoxorubicine,
chlorhydrate de 3'-désamino-3'-(N,N-diéthylformamidine)-épidoxorubicine, 3'-désamino-3'-(N,N-diéthylformamidine)-épidoxorubicine,
chlorhydrate de 3'-désamino-3'-(N,N-dipropylformamidine)-épidoxorubicine, 3'-désamino-3'-(N,N-dipropylformamidine)-épidoxorubicine,
chlorhydrate de 3'-désamino-3'-(N,N-dibutylformamidine)-épidoxorubicine, 3'-désamino-3'-(N,N-dibutylformamidine)-épidoxorubicine,
chlorhydrate de 3'-désamino-3'-[N-méthyl-N-(1"-phényléthyl)formamidine]-épidoxorubicine,
chlorhydrate de 3'-désamino-3'-(N,N-1",4"-tetraméthylèneacétamidine)-épidoxorubicine,
3'-désamino-3'-(N,N-1",4"-tetraméthyléneacétamidine)-épidoxorubicine,
chlorhydrate de 3'-désamino-3'-(N,N-3"-oxa-1",5"-pentaméihylèneacétamidine)-épidoxorubicine,
3'-désamino-3'-(N,N-3"-oxa-1",5"-pentaméthylèneacétamidine)-épidoxorubicine,
chlorhydrate de 3'-désamino-3'-(N,N,1",5"-pentaméthylèneacétamidine)-épidoxorubicine,
3'-désamino-3'-(N,N,1",5"-pentaméthylèneacétamidine)-épidoxorubicine,
chlorhydrate de 3'-désamino-3'-(N,N,1",6"-hexaméthylèneacétamidine)-épidoxorubicine,
chlorhydrate de 3'-désamino-3'-(N,N,1",7"-heptaméthyléneacétamidine)-épidoxorubicine,
chlorhydrate de 3'-désamino-3'-(N,N,3"-méthylaza-1",5"-pentaméthyléneacétamidine)-épidoxorubicine,
3'-désamino-3'-(N,N,3"-méthylaza-1",5"-pentaméthyléneacétamidine)-épidoxorubicine,
chlorhydrate de 3'-désamino-3'-(N,N-o-éthylènephényleneacétamidine)-épidoxorubicine,
3'-désamino-3'-(N,N-o-éthyléne-phényleneacétamidine)-épidoxorubicine,
chlorhydrate de 3'-désamino-3'-(N,N-diéthylacétamidine)-épidoxorubicine, 3'-désamino-3'-(N,N-diéthylacétamidine)-épidoxorubicine,
chlorhydrate de 3'-désamino-3'-(N,N-dipropylacétamidine)-épidoxorubicine, 3'-désamino-3'-(N,N-dipropylformamidine)-épidoxorubicine,
chlorhydrate de 3'-désamino-3'-(N,N-dibutylacétamidine)-épidoxorubicine, 3'-désamino-3'-(N,N-dibutylacétamidine)-épidoxorubicine,
chlorhydrate de 3'-désamino-3'-[N-méthyl-N-(1"-phényléthyl)acétamidine]-épidoxorubicine,
3'-désamino-3'-N,4'-O-méthylidene-daunorubicine,
3'-désamino-3'-N,4'-O-méthylidene-doxorubicine,
3'-désamino-3'-N,4'-O-éthylidene-daunorubicine,
3'-désamino-3'-N,4'-O-éthylidene-doxorubicine.

3. Utilisation selon la revendication 1, dans lequel antibiotiques anthracyclines sont inhibiteurs d'hélicase NS3 du virus de l'hépatite C (VHC) et sont utilisés pour la préparation d'un médicament pour inhibition de la réplication virale.

4. Utilisation selon la revendication 3, dans lequel antibiotiques anthracyclines sont administrés en dose suffisante pour inhibition de la virémie, causée par la réplication du virus de l'hépatite C.

5. Compositions pharmaceutiques comprenant des supports pharmaceutiquement acceptables, des diluants, des excipients et antibiotiques anthracyclines de la formule générale 1 ou 2, comme ingrédients actifs, où R¹ est hydrogène ou un groupe méthoxy, R² est hydrogène ou un groupe hydroxy, R³ est un groupe hydroxy dans une orientation axiale ou équatoriale, R⁴ et R⁵ sont des atomes d'hydrogène ou des groupes méthyles, R⁶ est un groupe 1-phényléthyle ou R⁵ et R⁶ avec un atome d'azote sont des groupes N,N-diéthyle, N,N-dipropyle ou N,N-dibutyle, ou R⁵ et R⁶ avec un atome d'azote forment N,N-1"4"-tetraméthyléne, N,N-3"-oxa-1",5"-pentaméthylène, N,N-1",5"-pentaméthylène, N,N-1",6"-hexaméthylène, N,N-1",7"-heptaméthylène, N,N-3"-méthylaza-1",5"-pentaméthylène ou un group 1-indolinyle, considérant que si X est une molécule de HCl, le composé est le chlorhydrate d'un dérivé de la formule générale 1 ou si X est O, le composé est un dérivé dans une forme de base libre de la formule générale 1 pour l'utilisation dans le traitement des infections causées par le virus de l'hépatite C.

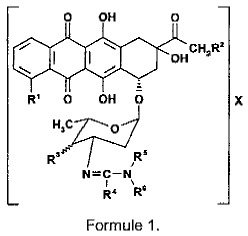

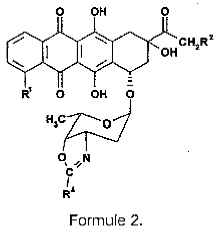

6. Les compositions pharmaceutiques pour l'utilisation selon la revendication 5, en combinaison avec un antibiotique anthracycline parent ou un autre médicament antiviral.
